# EUROPEAN PATENT SPECIFICATION

(11) **EP 3 581 182 B1**
(45) Date of publication and mention of the grant of the patent: **29.09.2021**
(21) Application number: 17718469.4
(22) Date of filing: 13.02.2017
(51) Int. Cl.: A61K 31/517, A61K 31/4166, A61P 35/00, A61K 31/4412, A61K 31/4439, A61K 31/496, A61K 31/5377

(54) **COMBINATION TREATING PROSTATE CANCER, PHARMACEUTICAL COMPOSITION AND TREATMENT METHOD**
KOMBINATIONSBEHANDLUNG FÜR PROSTATAKREBS, PHARMAZEUTISCHE ZUSAMMENSETZUNG UND BEHANDLUNGSVERFAHREN
ASSOCIATION MÉDICAMENTEUSE POUR LE TRAITEMENT DU CANCER DE LA PROSTATE, COMPOSITION PHARMACEUTIQUE ET MÉTHODE DE TRAITEMENT

(43) Date of publication of application: 18.12.2019
(73) Proprietor: Kangpu Biopharmaceuticals, Ltd., Shanghai 201203 (CN)
(72) Inventor: GE, Chuansheng, Shanghai 201203 (CN); LIAO, Baisong, Shanghai 201203 (CN); LEE, Wen-Cherng, Shanghai 201203 (CN)
(74) Representative: Kilburn & Strode LLP
(86) International application number: PCT/CN2017/073380
(87) International publication number: WO 2017/067530

(56) References cited:
- EP-A1- 3 020 714
- WO-A1-2013/087004
- WO-A1-2014/152833
- WO-A2-2015/160845
- CA-A1- 2 966 038
- US-A1- 2016 058 872
- PANG, RAN et al.: "Advances of Therapeutic Approaches for Hormone Refractory Prostate Cancer", CHINESE JOURNAL OF SURGERY , OF INTEGRATED TRADUTIONAL AND WESTERN MEDICINE, vol. 19, 28 February 2013 (2013-02-28), pages 100-102, XP009505371, ISSN: 1007-6948

## Description

### Field of invention

The present invention relates to a combination for the treatment of prostate cancer, a pharmaceutical composition and a treatment method.

### Prior arts

Prostate cancer is a common malignancy in the male reproductive system. The statistics which was made by the International Agency for Research on Cancer of World Health Organization in 2012 showed that the number of newly diagnosed prostate cancer patients in the world was 1.1 million in that year, accounting for about 15% of the total number of new cancer cases, making it the second most common cancer in men worldwide. In the United States, the incidence of prostate cancer ranks first in all malignancies, with the second highest mortality rate, second only to lung cancer. Although the incidence of prostate cancer in China is much lower than that in western countries, it has shown a significant growth trend in recent years and ranks first among male urological tumors, and most of prostate cancer were diagnosed in the terminal stage.

The growth of the prostate cancer cells requires the supporting of androgens including testosterone. Therefore, the targeted treatment strategies for prostate cancer mainly focus on the synthesis of androgen and the binding to the androgen receptor thereof. For example, Enzalutamide, a prostate cancer drug marketed by the U.S. FDA in August 2012, is a small molecule androgen receptor antagonist, which finally inhibits the androgen receptor pathway by competitive inhibition of the binding of androgen to its receptor, thereby achieving the effect of treating castration-resistant prostate cancer.

Enzalutamide also shows some side effects in clinical studies, such as weakness or fatigue, lumbago, diarrhea, joint pain, hot flashes, tissue swelling, musculoskeletal pain, headache, upper respiratory tract infection, dizziness, spinal cord compression and cauda equina syndrome, muscle weakness, dyscoimesis, lower respiratory tract infection, hematuria, tingling, anxiety and hypertension and so on.

CA2966038A1 discloses isoindoline compounds which are optionally combined with an androgen receptor inhibitor for use in the treatment of prostate cancer.

EP3020714A1 discloses imidazolidinediones as androgen receptor pathway modulators for use in the treatment of prostate cancer.

WO2015/160845A2 discloses benzoheterocyclyl compounds and conjugates to treat prostate cancer.

US2016/0058872A1 discloses a compound having the chemical structure comprising PTM-L-CLM wherein L is a linker group, CLM is a cereblon E3 Ubiquitin Ligase binding moiety, and PTM is a protein target moiety that binds to a bromodomain-containing protein or polypeptide, and wherein the PTM is chemically linked to the CLM through the linker group.

For the treatment of cancer, the drug combination is often used in the clinical practice to improve the treatment effect, for example, the combination of docetaxel and prednisone for use in the treatment of prostate cancer. However, people have met great setbacks when exploring new composition regimens. One of the typical examples is that although the composition of docetaxel and prednisone can treat prostate cancer (Tannock et al. N. Eng. J. Med. (2004), 351, 1502-1512), the combination regimen of docetaxel, prednisone and lenalidomide failed in a Phase III clinical trial involving more than 1000 prostate cancer patients (Petrylak et al. Lancet Oncol. (2015) 16-4, 417-425)*.* It should also be noted that, the results of several phase II clinical studies also indicated that the clinical efficacy of lenalidomide alone in the treatment of prostate cancer was not satisfying (Xing et al. Asian Pac. J. Cancer Prev. (2015) 16- 9, 3969-3972). Therefore, it has become an urgent technical problem to be solved in the art to explore composition regimens of anti-prostate cancer drugs (including Enzalutamide etc.) to improve the efficacy and reduce the toxic and side effect.

### Content of the present invention

The present invention provides a combination for the treatment of prostate cancer, a pharmaceutical composition and a treatment method. The combination for the treatment of prostate cancer, the pharmaceutical composition and the treatment method of the invention inhibit prostate cancer in a more effective manner.

The embodiments of the present invention are reflected in the independent claims. Preferred embodiments are depicted in the dependent claims.

The benzoheterocyclic compound is any one of the following compounds:

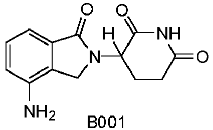

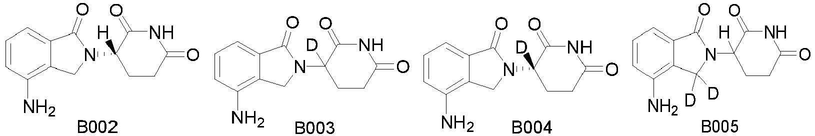

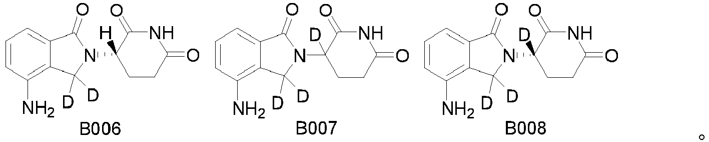

In the combination of the invention, preferably, the benzoheterocyclic compound is B001,.

In the combination of the invention, preferably, the androgen receptor pathway modulator is any one of the following compounds: AR1-1, AR1-2, AR2-1, AR2-2, AR2-3, AR2-4, AR2-5, AR3-1, AR3-2, AR3-3, AR3-4.

In some embodiments of the invention, the combination preferably comprises the benzoheterocyclic compound and the androgen receptor pathway modulator, wherein, the benzoheterocyclic compound is B001, the androgen receptor pathway modulator is selected from one of the AR1-1, AR1-2, AR1-3, AR1-4, AR2-1, AR2-2, AR2-3, AR2-4, AR2-5, AR2-6, AR2-7, AR2-8, AR2-9, AR2-10, AR2-11, AR2-12, AR2-13, AR2-14, AR2-15, AR2-16, AR2-17, AR2-18, AR2-19, AR2-20, AR2-21, AR2-22, AR2-23, AR2-24, AR2-25, AR2-26, AR2-27, AR3-1, AR3-2, AR3-3, AR3-4, AR3-5, AR3-6, AR3-7, AR3-8 and AR3-9; the androgen receptor pathway modulator is preferably selected from one of the AR1-1, AR1-2, AR2-1, AR2-2, AR2-3, AR2-4, AR2-5, AR3-1, AR3-2, AR3-3 and AR3-4.

In some embodiments of the invention, the combination is preferably any one of the following combinations: "B001 and AR1-1, "B001 and AR1-2, "B001 and AR1-3, "B001 and AR1-4, "B001 and AR2-1, "B001 and AR2-2, "B001 and AR2-3, "B001 and AR2-4, "B001 and AR2-5, "B001 and AR2-6, "B001 and AR2-7, "B001 and AR2-8, "B001 and AR2-9, "B001 and AR2-10, "B001 and AR2-11, "B001 and AR2-12, "B001 and AR2-13, "B001 and AR2-14, "B001 and AR2-15, "B001 and AR2-16, "B001 and AR2-17, "B001 and AR2-18, "B001 and AR2-19, "B001 and AR2-20, "B001 and AR2-21, "B001 and AR2-22, "B001 and AR2-23, "B001 and AR2-24, "B001 and AR2-25, "B001 and AR2-26, "B001 and AR2-27, "B001 and AR3-1, "B001 and AR3-2, "B001 and AR3-3, "B001 and AR3-4, "B001 and AR3-5, "B001 and AR3-6, "B001 and AR3-7, "B001 and AR3-8, "B001 and AR3-9.

The combination above, illustrated by the combination of "B001 and AR1-1, represents the combination of B001 and AR1-1. According to this explanation, one skilled in the art can understand the meanings of the other combinations mentioned above.

The combination of the invention may further comprise other therapeutic agents selected from one of Abiraterone, Abiraterone acetate, Galeterone, ODM201, Prednisone, Abiraterone and Prednisone, Abiraterone acetate and Prednisone, Galeterone and Prednisone, and, ODM201 and Prednisone.

Therefore, the combination of the invention preferably comprises the benzoheterocyclic compound , the androgen receptor pathway modulator and the other therapeutic agent, wherein, the benzoheterocyclic compound is B001,; the androgen receptor pathway modulator is selected from one of the AR1-1, AR1-2, AR1-3, AR1-4, AR2-1, AR2-2, AR2-3, AR2-4, AR2-5, AR2-6, AR2-7, AR2-8, AR2-9, AR2-10, AR2-11, AR2-12, AR2-13, AR2-14, AR2-15, AR2-16, AR2-17, AR2-18, AR2-19, AR2-20, AR2-21, AR2-22, AR2-23, AR2-24, AR2-25, AR2-26, AR2-27, AR3-1, AR3-2, AR3-3, AR3-4, AR3-5, AR3-6, AR3-7, AR3-8 and AR3-9; the androgen receptor pathway modulator is preferably selected from one of the AR1-1, AR1-2, AR2-1, AR2-2, AR2-3, AR2-4, AR2-5, AR3-1, AR3-2, AR3-3, AR3-4; the other therapeutic agent is selected from one of Abiraterone, Abiraterone acetate, Galeterone, ODM201, Prednisone, Abiraterone and Prednisone, Abiraterone acetate and Prednisone, Galeterone and Prednisone, and, ODM201 and Prednisone.

In some embodiments of the invention, the combination is preferably any one of the following combinations: "B001 and AR1-1 and Abiraterone, "B001 and AR1-2 and Abiraterone, "B001 and AR2-1 and Abiraterone, "B001 and AR2-2 and Abiraterone, "B001 and AR2-3 and Abiraterone, "B001 and AR2-4 and Abiraterone, "B001 and AR2-5 and Abiraterone, "B001 and AR3-1 and Abiraterone, "B001 and AR3-2 and Abiraterone, "B001 and AR3-3 and Abiraterone, "B001 and AR3-4 and Abiraterone, "B001 and AR1-1 and Abiraterone acetate, "B001 and AR1-2 and Abiraterone acetate, "B001 and AR2-1 and Abiraterone acetate, "B001 and AR2-2 and Abiraterone acetate, "B001 and AR2-3 and Abiraterone acetate, "B001 and AR2-4 and Abiraterone acetate, "B001 and AR2-5 and Abiraterone acetate, "B001 and AR3-1 and Abiraterone acetate, "B001 and AR3-2 and Abiraterone acetate, "B001 and AR3-3 and Abiraterone acetate, "B001 and AR3-4 and Abiraterone acetate, "B001 and AR1-1 and Galeterone, "B001 and AR1-2 and Galeterone, "B001 and AR2-1 and Galeterone, "B001 and AR2-2 and Galeterone, "B001 and AR2-3 and Galeterone, "B001 and AR2-4 and Galeterone, "B001 and AR2-5 and Galeterone, "B001 and AR3-1 and Galeterone, "B001 and AR3-2 and Galeterone, "B001 and AR3-3 and Galeterone, "B001 and AR3-4 and Galeterone, "B001 and AR1-1 and ODM201, "B001 and AR1-2 and ODM201, "B001 and AR2-1 and ODM201, "B001 and AR2-2 and ODM201, "B001 and AR2-3 and ODM201, "B001 and AR2-4 and ODM201, "B001 and AR2-5 and ODM201, "B001 and AR3-1 and ODM201, "B001 and AR3-2 and ODM201, "B001 and AR3-3 and ODM201, "B001 or and AR3-4 and ODM201, "B001 and AR1-1 and Prednisone, "B001 and AR1-2 and Prednisone, "B001 and AR2-1 and Prednisone, "B001 and AR2-2 and Prednisone, "B001 and AR2-3 and Prednisone, "B001 and AR2-4 and Prednisone, "B001 and AR2-5 and Prednisone, "B001 and AR3-1 and Prednisone, "B001 and AR3-2 and Prednisone, "B001 and AR3-3 and Prednisone, "B001 and AR3-4 and Prednisone, "B001 and AR1-1 and Abiraterone and Prednisone, "B001 and AR1-2 and Abiraterone and Prednisone, "B001 and AR2-1 and Abiraterone and Prednisone, "B001 and AR2-2 and Abiraterone and Prednisone, "B001 and AR2-3 and Abiraterone and Prednisone, "B001 and AR2-4 and Abiraterone and Prednisone, "B001 and AR2-5 and Abiraterone and Prednisone, "B001 and AR3-1 and Abiraterone and Prednisone, "B001 and AR3-2 and Abiraterone and Prednisone, "B001 and AR3-3 and Abiraterone and Prednisone, "B001 and AR3-4 and Abiraterone and Prednisone, "B001 and Abiraterone acetate and Prednisone, "B001 and AR1-2 and Abiraterone acetate and Prednisone, "B001 or F001 or K001 or D108" and AR2-1 and Abiraterone acetate and Prednisone, "B001 and AR2-2 and Abiraterone acetate and Prednisone, "B001 and AR2-3 and Abiraterone acetate and Prednisone, "B001 and AR2-4 and Abiraterone acetate and Prednisone, "B001 and AR2-5 and Abiraterone acetate and Prednisone, "B001 and AR3-1 and Abiraterone acetate and Prednisone, "B001 and AR3-2 and Abiraterone acetate and Prednisone, "B001 and AR3-3 and Abiraterone acetate and Prednisone, "B001 and AR3-4 and Abiraterone acetate and Prednisone, "B001 and AR1-1 and Galeterone and Prednisone, "B001 and AR1-2 and Galeterone and Prednisone, "B001 and AR2-1 and Galeterone and Prednisone, "B001 and AR2-2 and Galeterone and Prednisone, "B001 and AR2-3 and Galeterone and Prednisone, "B001 and AR2-4 and Galeterone and Prednisone, "B001 and AR2-5 and Galeterone and Prednisone, "B001 and AR3-1 and Galeterone and Prednisone, "B001 and AR3-2 and Galeterone and Prednisone, "B001 and AR3-3 and Galeterone and Prednisone, "and AR3-4 and Galeterone and Prednisone, "B001 and AR1-1 and ODM201 and Prednisone, "B001 and AR1-2 and ODM201 and Prednisone, "B001 and AR2-1 and ODM201 and Prednisone, "B001 and AR2-2 and ODM201 and Prednisone, "B001 and AR2-3 and ODM201 and Prednisone, "B001 and AR2-4 and ODM201 and Prednisone, "B001 and AR2-5 and ODM201 and Prednisone, "B001 and AR3-1 and ODM201 and Prednisone, "B001 and AR3-2 and ODM201 and Prednisone, "B001 and AR3-3 and ODM201 and Prednisone, "B001 and AR3-4 and ODM201 and Prednisone.

The combination above, illustrated by the combination of "B001 and AR1-1 and Abiraterone, represents the combination of B001 and AR1-1 and Abiraterone,. According to this explanation, one skilled in the art can understand the meanings of the other combinations mentioned above.

Each of the components in the combination may be administered simultaneously or separately (eg, sequentially). When the components in the combination are administered simultaneously, the components in the combination may be uniformly mixed (ie, the mixture of components).

In the invention, the term "component" refers to a component in the combination of the invention, that is one of the benzoheterocyclic compound, the pharmaceutically acceptable salt, solvate, polymorph, co-crystal, stereoisomer and isotope compound, thereof, the androgen receptor pathway modulator or the other therapeutic agents.

The present invention further provides a pharmaceutical composition, comprising the above combination and one or more of the pharmaceutically acceptable excipients.

In one aspect, the pharmaceutical composition of the invention may comprise one of the benzoheterocyclic compound , the pharmaceutically acceptable salt, solvate, polymorph, co-crystal, stereoisomer and isotope compound, thereof as mentioned above, the androgen receptor pathway modulator as mentioned above, and one or more of the pharmaceutically acceptable excipients.

In another aspect, the pharmaceutical composition of the invention may comprise one of the benzoheterocyclic compound, the pharmaceutically acceptable salt, solvate, polymorph, co-crystal, stereoisomer and isotope compound, thereof as mentioned above, the androgen receptor pathway modulator as mentioned above, the other therapeutic agents as mentioned above, and one or more of the pharmaceutically acceptable excipients.

The pharmaceutically acceptable excipients can be those widely used in drug manufacture field. The excipient is mainly used to provide a safe, stable and functionalized pharmaceutical composition, and can also provide a method which makes the active ingredients dissolved at a desired rate after the subject receives administration or promotes the efficacy of absorption of the active ingredients after the subject is administered with the composition. The excipient can be an inert filler, or provide a certain function, such as stabilizing the overall pH value of the composition or preventing the degradation of the active ingredients of the composition. The pharmaceutically acceptable excipient may comprise one or more of the following excipients: binder, suspending agent, emulsifier, diluent, filler, granulating agent, adhesive, disintegrating agent, lubricant, anti-adhesive agent, glidant, wetting agent, gelling agent, absorption retarder, dissolution inhibitor, reinforcing agent, adsorbent, buffer, chelating agent, preservative, colorant, flavoring agent and sweetening agent.

The methods of preparing pharmaceutical compositions known to people skilled in the art include but not limited to conventional mixing, dissolving, granulating, emulsifying, grinding, encapsulating, embedding or lyophilization. For example, the pharmaceutical composition of the present invention can be prepared by mixing one of the benzoheterocyclic compound, the pharmaceutically acceptable salt, solvate, polymorph, co-crystal, stereoisomer, isotope compound, metabolite and prodrug thereof, the androgen receptor pathway modulator and the pharmaceutically acceptable excipient, or by mixing one or more of the benzoheterocyclic compound, the pharmaceutically acceptable salt, solvate, polymorph, co-crystal, stereoisomer and isotope compound thereof, the androgen receptor pathway modulator, the other therapeutic agents and the pharmaceutically acceptable excipient.

The pharmaceutical composition of the invention may be formulated into any form for administration, including injection (intravenous), mucosal, oral administration (solid and liquid preparation), inhalation, ocular administration, rectal administration, topical or parenteral (infusion, injection, implantation, subcutaneous, vein, artery, intramuscular) administration. The pharmaceutical composition of the present invention can also be a controlled release or delayed release preparation. Examples of solid oral preparations include but not limited to powder, capsule, caplet, soft capsule, pill and tablet. Examples of liquid preparations for oral or mucosal administration include but not limited to suspension, emulsion, elixir and solution. Examples of preparations for topical administration include but not limited to emulsion, gel, ointment, cream, patch, paste, foam, lotion, drops or serum preparation. Examples of preparations for parenteral administration include but not limited to injection solution, dry preparation which can be dissolved or suspended in a pharmaceutically acceptable carrier, injection suspension and injection emulsion. Examples of other suitable preparations of the pharmaceutical composition, include but not limited to eye drops and other ophthalmic preparations; aerosol, such as nasal spray or inhalation; liquid dosage forms suitable for parenteral administration; suppository and pastille.

In another aspect, the invention provides a kit, comprising pharmaceutical composition A and pharmaceutical composition B;

The pharmaceutical composition A comprises the benzoheterocyclic compound used in the invention, the pharmaceutically acceptable salt, solvate, polymorph, co-crystal, stereoisomer and isotope compound thereof as mentioned above and one or more of the pharmaceutically acceptable excipients; The pharmaceutical composition B comprises the androgen receptor pathway modulator as mentioned above and one or more of the pharmaceutically acceptable excipients.

The kit may further comprise a pharmaceutical composition C, which comprises the other therapeutic agents as mentioned above and one or more of the pharmaceutically acceptable excipients.

Preferably, the benzoheterocyclic compound, the pharmaceutically acceptable salt, solvate, polymorph, co-crystal, stereoisomer and isotope compound thereof in the pharmaceutical composition A, the androgen receptor pathway modulator in the pharmaceutical composition B, the other therapeutic agents in the pharmaceutical composition C and the combination thereof are as mentioned above.

The pharmaceutical compositions in the kit may be administered simultaneously or separately (eg, sequentially).

In the kit, the term "pharmaceutically acceptable excipients" has the same definition as above.

In the invention, the term "active ingredient" refers to the active ingredient in the pharmaceutical composition or the kit of the invention, that is, one of the benzoheterocyclic compound mentioned above, the pharmaceutically acceptable salt, solvate, polymorph, co-crystal, stereoisomer and isotope compound thereof, the androgen receptor pathway modulator, the other therapeutic agents or the combination thereof.

The above combination, the above pharmaceutical composition or the above kit can be used for the prevention and/or treatment of prostate cancer. The prostate cancer is preferably castration-resistant prostate cancer.

In another aspect, the invention provides a use of the above one of the benzoheterocyclic compound, the pharmaceutically acceptable salt, solvate, polymorph, co-crystal, stereoisomer and isotope compound thereof, in the manufacture of a medicament for the prevention and/or treatment of prostate cancer in combination with the above androgen receptor pathway modulator.

The invention provides a use of the above androgen receptor pathway modulator, in the manufacture of a medicament for the prevention and/or treatment of prostate cancer in combination with the above one of the benzoheterocyclic compound, the pharmaceutically acceptable salt, solvate, polymorph, co-crystal, stereoisomer and isotope compound thereof.

In another aspect, the invention provides a use of the above one of the benzoheterocyclic compound, the pharmaceutically acceptable salt, solvate, polymorph, co-crystal, stereoisomer and isotope compound thereof, in the manufacture of a medicament for the prevention and/or treatment of prostate cancer in combination with the above androgen receptor pathway modulator and the above other therapeutic agents.

The invention provides a use of the above androgen receptor pathway modulator, in the manufacture of a medicament for the prevention and/or treatment of prostate cancer in combination with the above one of the benzoheterocyclic compound, the pharmaceutically acceptable salt, solvate, polymorph, co-crystal, stereoisomer and isotope compound thereof and the above other therapeutic agents.

The invention provides a use of the above other therapeutic agents, in the manufacture of a medicament for the prevention and/or treatment of prostate cancer in combination with the above one of the benzoheterocyclic compound, the pharmaceutically acceptable salt, solvate, polymorph, co-crystal, stereoisomer and isotope compound thereof and the above androgen receptor pathway modulator.

In the use of the invention, the above one of the benzoheterocyclic compound , the pharmaceutically acceptable salt, solvate, polymorph, co-crystal, stereoisomer and isotope compound thereof, the above androgen receptor pathway modulator and the above other therapeutic agents may be administered simultaneously or separately (eg, sequentially).

In the use, the one of the benzoheterocyclic compound, the pharmaceutically acceptable salt, solvate, polymorph, co-crystal, stereoisomer and isotope compound thereof, the androgen receptor pathway modulator, the other therapeutic agent and the combination thereof are as described above.

In another aspect, the invention describes a method of prevention and/or treatment of prostate cancer, comprising administration of a therapeutically or prophylactically effective amount of the above combination to the patients in need. The prostate cancer can be castration-resistant prostate cancer.

The method of prevention and/or treatment of prostate cancer, comprises administration of a therapeutically or prophylactically effective amount of the above one of benzoheterocyclic compound, the pharmaceutically acceptable salt, solvate, polymorph, co-crystal, stereoisomer and isotope compound thereof and a therapeutically or prophylactically effective amount of the above androgen receptor pathway modulator to the patients in need.

Wherein, the above one of the benzoheterocyclic compound, the pharmaceutically acceptable salt, solvate, polymorph, co-crystal, stereoisomer and, isotope compound thereof and the above androgen receptor pathway modulator may be administered simultaneously or separately (eg, sequentially).

The method of prevention and/or treatment of prostate cancer preferably comprises administration of a therapeutically or prophylactically effective amount of the above one of the benzoheterocyclic compound, the pharmaceutically acceptable salt, solvate, polymorph, co-crystal, stereoisomer and isotope compound thereof, a therapeutically or prophylactically effective amount of the above androgen receptor pathway modulator and a therapeutically or prophylactically effective amount of the above other therapeutic agent to the patients in need.

Wherein, the above one of the benzoheterocyclic compound , the pharmaceutically acceptable salt, solvate, polymorph, co-crystal, stereoisomer and isotope compound thereof, the above androgen receptor pathway modulator and the above other therapeutic agent may be administered simultaneously or separately (eg, sequentially).

In the method of prevention and/or treatment of prostate cancer, the one of the benzoheterocyclic compound, the pharmaceutically acceptable salt, solvate, polymorph, co-crystal, stereoisomer and isotope compound thereof, the androgen receptor pathway modulator, the other therapeutic agent and the combination thereof are as described above.

In the combination, the pharmaceutical composition, the kit, the use or the method of prevention and/or treatment of prostate cancer of the invention, the mole ratio of the benzoheterocyclic compound and the androgen receptor pathway modulator, can be selected in accordance with the conventional art, for example, 1:0.1-1:100, for example, 1:1-1:50, for example, 1:1-1:10.

In the combination, the pharmaceutical composition, the kit, the use or the method of prevention and/or treatment of prostate cancer of the invention, when the other therapeutic agent is further comprised, the amount of the other therapeutic agent is not particularly limited, for example, the mole ratio of the other therapeutic agent and the androgen receptor pathway modulator can be 1:0.1-1:100, for example, 1:1-1:50, for example, 1:1-1:10.

In the combination, the pharmaceutical composition, the kit, the use or the method of prevention and/or treatment of prostate cancer of the invention, the amount of the benzoheterocyclic compound and the androgen receptor pathway modulator can be selected in accordance with the conventional art, for example, the amount of the benzoheterocyclic compound can be 1-100µM, for example, 1-50µM, for example, 1-10µM; the amount of the androgen receptor pathway modulator can be 1-300µM, for example, 1-100µM, for example, 1-10µM.

In the combination, the pharmaceutical composition, the kit, the use or the method of prevention and/or treatment of prostate cancer of the invention, when the other therapeutic agent is further comprised, the amount of the other therapeutic agent is not particularly limited, for example, the amount of the other therapeutic agent can be 1-300µM, for example, 1-100µM, for example, 1-10µM.

When each of the components in the combination of the invention is administered to a subject for the purpose of treating or preventing a disease, disorder or condition, each component in the combination may be administered by the same route or by a different route. The route of administration may be any route described herein, including but not limited to oral, inhalation, injection, ophthalmic, mucosal, rectal, emulsion, liposome, long-acting implant or sustained controlled release method. The specific route of administration will depend on the therapeutic agent itself and the preparation, as well as the disease, disorder or condition to be prevented or treated. According to the present disclosure, the skill level of an ordinary person skilled in the art is sufficient to determine the route of administration. Each of the components in the combination of the invention may be administered to the subject within a period of time (administration period) followed by a period of no administration of the compound (non-administration period). The administration period and non-administration period can be repeated for desired times. The desired length and times of the administration period or non-administration period will depend on the type and/or severity of the disease, disorder or condition being treated or prevented, as well as the sex, age, weight, and other parameters (e.g. the individual subject's biological, physical, and physiological status, etc.) of the individual subject. Each of the components in the combination of the invention may be administered simultaneously to the subject in a period of time and may also be administered to the subject sequentially in a period of time. According to the present disclosure, the skill level of an ordinary person skilled in the art is sufficient to determine the appropriate length and times of administration period and / or non-administration period.

The therapeutic method comprises administering each of the components in the combination of the invention to a subject by any suitable methods, such as injection, mucosal, oral, inhalation, ocular, rectal, long-acting implant, liposome, emulsion or sustained release process.

One skilled in that the art will understand that the therapeutically or prophylactically effective amount of each of the components or the active ingredients in the combination, pharmaceutical composition or the kit of the invention may vary with factors, for a specific subject, such as age, diet, health, etc., the symptom or disease to be treated or prevented, the severity of the disorder or condition, and the complications and types, and the preparations used etc. According to the disclosures in the invention, one skilled in the art can easily determine the desired therapeutically or prophylactically effective amount administered to the subject, so as to induce the desired biological or medical response in the subject.

In another embodiment, the combination, the pharmaceutical composition, the kit, the use or the method of prevention and/or treatment of prostate cancer of the invention, the therapeutically or prophylactically effective amount of the androgen receptor pathway modulator or the other therapeutic agent may be lower than the effective amount when the benzoheterocyclic compound, the pharmaceutically acceptable salt, solvate, polymorph, co-crystal, stereoisomer and isotope compound thereof of the present invention is not administered.

In the invention, the amount of the compound administered, the therapeutically or prophylactically effective amount, the dosage, the starting dosage and the like are all referred to the amount of a specific compound, for example, a specific heterocyclic compound, the pharmaceutically acceptable salt, solvate, polymorph, co-crystal, stereoisomer or isotope compound thereof, a specific androgen receptor pathway modulator or a specific other therapeutic agent, rather than a combination of multiple compounds.

In the method, the therapeutically or prophylactically effective amount of the androgen receptor pathway modulator or the other therapeutic agent and the guidance for administration can be found in the patents and published patent applications cited herein, and Wells et al, eds., Pharmacotherapy Handbook, 2nd Edition, Appleton and Lange, Stamford, Conn. (2000); PDR Pharmacopoeia, Tarascon Pocket Pharmacopoeia 2000, Deluxe Edition, Tarascon Publishing, Loma Linda, Calif. (2000) and other medical literatures.

In some embodiments, the therapeutically or prophylactically effective amount of the compound (herein referred as to one of the benzoheterocyclic compound , the pharmaceutically acceptable salt, solvate, polymorph, co-crystal, stereoisomer and isotope compound thereof, the androgen receptor pathway modulator, or the other therapeutic agent) administered to each subject is from about 0.005 to about 1000 mg/day, from about 0.01 to about 500 mg/day, from about 0.01 to about 250 mg/day, from about 0.01 to about 100 mg/day, from about 0.1 to about 100 mg/day, from about 0.5 to about 100 mg/day, from about 1 to about 100 mg/day, from about 0.01 to about 50 mg/day, from about 0.1 to about 50 mg/day, from about 0.5 to about 50 mg/day, from about 1 to about 50 mg/day, from about 0.02 to about 25 mg/day, or from about 0.05 to about 10 mg/day.

In some embodiments, the therapeutically or prophylactically effective amount (herein referred as to the therapeutically or prophylactically effective amount of the one of the benzoheterocyclic compound, the pharmaceutically acceptable salt, solvate, polymorph, co-crystal, stereoisomer and isotope compound thereof, the androgen receptor pathway modulator, or the other therapeutic agent) is about 0.01, about 0.05, about 0.1, about 0.2, about 0.3, about 0.4, about 0,5, about 0.6, about 0.8, about 1, about 2, about 5, about 10, about 15, about 20, about 25, about 30, about 40, about 45, about 50, About 60, about 70, about 80, about 90, about 100, about 150, about 200, about 250, about 300, about 350, about 400, about 450, about 500, about 550, about 600, about 650, about 700 About 750, about 800, about 850, about 900, or about 1000 mg/day/subject.

In any of the methods described herein, including but not limited to the above therapeutic methods, applications etc., the combination, the pharmaceutical composition or the kit according to the invention may be used alone or in combination with ultrasound therapy, radiation therapy (referred to as radiotherapy) or radioimmunotherapy etc., and may also be used in combination with one or more of other pharmacologically active therapeutic agents (hereinafter referred to as "other active agents"). The amount and type of other active agents will depend on the disease, disorder or condition to be treated or prevented; the severity of the disease, disorder or condition; factors of the subject administrated with the composition, such as age, weight, physical conditions, etc.; the route of administration, and so on. According to the embodiments of the invention, the other active agent may be a natural, semi-synthetic or synthetic compound. In another embodiment, the other therapeutic agent may be a small molecule, such as a synthetic organic or inorganic molecule; or a larger molecule or biomolecule, such as a protein or nucleic acid with pharmacological activity. In another embodiment, the other therapeutic agent may be one or more of a chemotherapeutant, an antiangiogenic drug (also known as an angiogenesis inhibitor), an immunomodulatory agent, an immunotherapeutic agent, a monoclonal antibody, a polyclonal antibody, and a kinase inhibitor.

The chemotherapeutant (chemotherapeutic agent), is a chemically synthesized drug. Currently, the chemotherapeutant is the main drug in the treatment of cancer and some autoimmune diseases, what commonly used are: epirubicin, doxorubicin, daunorubicin, mitomycin, fluorouracil deoxynucleotides and so on.

The antiangiogenic drug inhibits angiogenesis by inhibiting pro-angiogenic growth factor, growth factor receptor or signaling pathway downstream etc., so as to inhibit the growth and metastasis of the tumors, and it mainly includes vascular endothelial growth inhibitor, receptor tyrosine kinase inhibitor, PI3K/AKT/mTOR pathway inhibitor, recombinant fusion protein (e.g. aflibercept) acting on VEGF-A, VEGF-B and placental growth factor, recombinant human endostatin and so on.

The immunomodulatory agent is a drug which can enhance, promote and regulate immune functions and have a certain effect on immune dysfunction, some secondary immunodeficiency diseases and some malignant tumors. In accordance with the functions of the immunomodulatory agent, the immunomodulatory agent is mainly divided into immunosuppressant and immunopotentiator. The former is used for anti-inflammatory, anti-autoimmune reactions, anti-allergy, anti-transplant rejection and anti-tumor, and the latter is for anti-infection, anti-allergy, and anti-tumor. Various kinds of drugs belong to immunosuppressant, including antimetabolic drugs(cyclosporin A, azathioprine, cyclophosphamide, methotrexate, mycophenolate, tacrolimus, mizoribine etc.), glucocorticoid, monoclonal antibody (anti-TNF-alpha/receptor, anti-IFN-y, and anti-CD25 monoclonal antibody, etc.), cytokines IFN-β, IL-10 and TGF-β, chemicals(leflunomide and 5-HT3 receptor antagonist), non-steroid anti-inflammatory drugs, nucleic acids, statins anti-lipid drugs, HMG coenzyme A reductase inhibitor, plants (Tripterygium wilfordii, extract of Cordyceps sinensis FTY720, artemisinin and Parviline etc.) and other biological products (cholera toxin B subunit, sNTB-A-Fc fusion protein, CMV-IkappaBa carrier inhibitor and B7-HI inhibitor etc.). There are also various kinds of immunopotentiators, including cytokines(interferon α, interferon γ, thymic peptide, Thymopentin, G-CSF/GM-CSF, IL-2, IL-12, recombinant human erythropoietin, epidermal growth factor, chemokine intercellular adhesion molecule-1, vascular cell adhesion molecule-1, P-selectin, and other intercellular adhesion molecules, etc.), biological products [IVIG, transfer factor, immune riboncleic acid, bacteria and its extract (Bacillus Calmette Guerin and its extract, defatted and deactivated mycobacterium vaccine, other bacterial extracts, low calcium response V or V antigen LcrV, vibrio cholerae products Zot and mycobacterium etc.)], plant drugs (polysaccharides, saponins and other plant ingredients), chemicals (Levamisole, Tagamet, Pidotimod, NS-398 Imiquimod, Propagermanium and liposome etc.), micronutrients (vitamin A/C/D, trace elements iron, zinc, selenium) and others (macrolide antibiotics, aminophylline).

Immunotherapy refers to the modulation of the immune response of a subject to produce the desired therapeutic effect, the immunotherapeutic agent refers to a drug that when administered to a subject modulates the immune system of the subject so as to be sufficient to ultimately reduce the symptoms associated with an adverse immune response or ultimately reduce the symptoms caused by the increase of the required immune response.

The monoclonal antibody refers to a highly uniform antibody, produced by a single B cell clone, targeting only a specific epitope.

The polyclonal antibody refers to different antibodies produced by using an antigen immune receptor that contains multiple antigenic determinants to stimulate multiple B cell clones in the body, targeting multiple antigenic epitopes.

In biochemistry, kinases are enzymes that transfer phosphate groups from high-energy donor molecules (such as ATP) to specific target molecules (substrates); and this process is called phosphorylation; the kinase inhibitor refers to a class of molecules that may bind with kinases and reduce their activity.

The other active agent includes but not limited to daratumumab, elotuzumab, palbociclib, panobinostat, nivolumab, pembrolizumab, pemetrexed, topotecan, doxorubicin, bortezomib, gemcitabine, dacarbazine, biaxin, vincristine, azacitidine, CAR-T, rituximab, trastuzumab, PD-1 inhibitor, PD-L1 inhibitor, HDAC inhibitor, androgen receptor pathway regulators other than the aforementioned androgen receptor pathway regulators, docetaxel, clofarabine injection, Ublituximab, romidepsin, BTK inhibitor, erythropoietin, eltrombopag, minocycline and melphalan.

The term "androgen receptor pathway modulator" in the invention comprises androgen inhibitor, androgen receptor inhibitor, androgen biosynthesis inhibitor and other drugs that affect the androgen receptor pathway.

The term "hormones" are a class of chemicals that are produced by certain tissues of a normal body, and then diffuse into the blood, and are transported to other tissues in the body by blood circulation to exert special physiological functions. Hormonal compounds include synthetic or natural hormonal chemicals.

As used herein, when referring to a specific salt, composition, and excipient etc. as "pharmaceutically acceptable", it means that the salt, the composition, the excipient etc. are generally non-toxic, safe, and suitable for use in a subject, preferably a mammalian subject, more preferably a human subject.

The term "pharmaceutically acceptable salt" herein refers to a pharmaceutically acceptable organic or inorganic salt. Examples of the salt include but are not limited to: sulfate, citrate, acetate, oxalate, chloride, bromide, iodide, nitrate, hydrosulfate, phosphate, acid phosphate, isonicotinic acid salt, lactate, salicylic acid salt, acid citrate, tartrate, oleate, tannate, pantothenate, bitartrate, ascorbate, succinate, maleate, gentisinate, fumarate, gluconate, glucuronate, saccharate, formate, benzoate, glutamate, methane sulfonate, ethane sulfonate, benzene sulfonate, p-toluene sulfonate, and embonate (i.e. 1-1-methylene-bis(2-hydroxy-3-naphthoate)). The compounds of the present invention may form pharmaceutically acceptable salts with various amino acids. Suitable alkali salts include but are not limited to, aluminum salt, calcium salt, lithium salt, magnesium salt, potassium salt, sodium salt, zinc salt, bismuth salt and diethanolamine salt. For a review of the pharmaceutically acceptable salts, see Handbook of Pharmaceutical Salts: Properties, Selection, and Use (P. Heinrich Stahl and Camille G. Wermuth, ed., Wiley-VCH, 2002).

As used herein, the term "metabolite" refers to an active substance produced by changes in chemical structure that a drug molecule undergoes in vivo, the active substance is generally a derivative of the aforementioned drug molecule, and can also be chemically modified.

As used herein, the term "polymorph" refers to one or more crystal structures formed by the different arrangement of molecules in the lattice space when crystallized.

As used herein, the term "co-crystal" refers to a multi-component system comprising one or more API (active pharmaceutical ingredient) molecules and one or more object (or ligand) molecules. In the co-crystal, API molecules and object (or ligand) molecules exist as solids at room temperature when they are used as their pure form alone (in order to distinguish co-crystal from solvate or hydrate). From this particular definition, salts in which significant or complete proton exchange occurs between API molecules and guest molecules are excluded. In the co-crystal, API and ligands interact through hydrogen bonds and other possible non-covalent interactions. It is noted that the co-crystal itself may form solvates, including hydrates. The object (or ligand) refers to other physiologically acceptable acids, bases or non-ionic compounds.

As used herein, the term "solvate" refers to a crystal form of the benzoheterocyclic compound used according to the invention, , the pharmaceutically acceptable salt, polymorph, co-crystal, stereoisomer, isotopic compound, metabolite or prodrug thereof, which further comprises one or more solvent molecule(s) incorporated into the crystal structure. The solvate may include a stoichiometric amount or a non-stoichiometric amount of solvent, and the solvent molecule in the solvent may exist in an ordered or non-ordered arrangement. The solvate containing a non-stoichiometric amount of solvent molecules may be obtained by the loss of at least one solvent molecule (but not all) from the solvate. In a particular embodiment, a solvate refers to a hydrate, which means the crystal of the compound further comprises water molecules, with water molecules as the solvent.

As used herein, the term "prodrug" refers to a derivative of the compound comprising a biologically reactive functional group such that the biological reactive functional group can be cleaved from the compound or react in other ways to give the compound under biological conditions (in vivo or in vitro). Usually, the prodrug is inactive, or at least has lower activity than the compound itself, so that the compound exhibits its activity until it is cleaved from the biologically reactive functional group. The biologically reactive functional group can be hydrolyzed or oxidized under biological conditions to give the compound. For instance, the prodrug may contain a biologically hydrolysable group. Examples of the biologically hydrolysable group include but are not limited to: a biologically hydrolysable phosphate, a biologically hydrolysable ester, a biologically hydrolysable amide, a biologically hydrolysable carbonic ester, a biologically hydrolysable carbamate and a biologically hydrolysable ureide. For a review of the prodrug, see, for example, J. Rautio et al., Nature Reviews Drug Discovery (2008) 7, 255-270 and Prodrugs: Challenges and Rewards (V. Stella et al. ed., Springer, 2007).

The benzoheterocyclic compound, the pharmaceutically acceptable salt, solvate, polymorph, co-crystal, stereoisomer and, isotope compound thereof in the combination of the invention, may contain one or more asymmetric centers ("stereoisomer"). As used herein, the term "stereoisomer" refers to all stereoisomers including enantiomers, diastereoisomers, epimers, endo-exo isomers, atropisomers, regioisomers, cis- and trans-isomers. The "stereoisomer" herein also includes "pure stereoisomer" and "enriched stereoisomer" or "racemic isomer" of the various aforementioned stereoisomers. These stereoisomers can be prepared according to an asymmetric synthesis process, or separated, purified and enriched by a chiral separation process (including but not limited to thin layer chromatography, rotating chromatography, column chromatography, gas chromatography, high pressure liquid chromatography, etc.), and can also be obtained through chiral separation by means of bonding (chemical binding etc.) or salifying (physical binding etc.) with other chiral compound(s). The term "pure stereoisomer" herein refers to a stereoisomer of the compound with the mass content of no less than 95% relative to other stereoisomers of the compound. The term "enriched stereoisomer" herein refers to a stereoisomer of the compound with the mass content of no less than 50% relative to other stereoisomers of the compound. The term "racemic isomer" herein refers to a stereoisomer of the compound with the mass content equal to that of other stereoisomers of the compound.

As used herein, D represents deuterium-enriched hydrogen, and H represents non-deuterium-enriched hydrogen. "Deuterium-enriched" compound means that abundance of deuterium at any relevant site in the benzoheterocyclic compound, the pharmaceutically acceptable salt, solvate, polymorph, co-crystal, stereoisomer or isotope compound thereof is greater than its natural abundance at that site (0.0156%). So, in the "deuterium-enriched" compounds, the abundance of deuterium at any of its related sites may be in the range of 0.0156% to 100%. An example of a process for obtaining deuterium-enriched compounds is to exchange hydrogen with deuterium or to synthesize the compound from deuterium-enriched starting material.

Based on the general knowledge in the art, the symbol H may be omitted in the non-deuterium-enriched site. "Non-deuterium enriched" refers to hydrogen in nature, i.e., in the form of isotopic mixture of H (hydrogen or protium), D (²H or deuterium) and T (³H or tritium).

The term "isotopic compound" used herein refers to the benzoheterocyclic compound of, the pharmaceutically acceptable salt, the solvate, the polymorph, the co-crystal, the stereoisomer or the isotopic compound thereof containing one or more atomic isotope(s) with natural or non-natural abundance. Atomic isotopes with non-natural abundance include but are not limited to deuterium (²H or D), tritium (³H or T), iodine-125 (¹²⁵I), phosphorus-32 (³²P), carbon-13 (¹³C) or carbon-14 (¹⁴C). The aforementioned isotopic compound can also be used as a therapeutic or diagnostic agent (i.e., internal developing agent) or a research tool. All the isotopic variants of the compound of the present invention, whether radioactive or not, are included in the scope of the present invention.

The term "isotope enriched" used herein refers to the benzoheterocyclic compound, the pharmaceutically acceptable salt, solvate, polymorph, co-crystal or stereoisomer thereof containing one or more atomic isotope(s) with non-natural abundance. The term "isotope enriched" also refers to the heterocyclic compound, the pharmaceutically acceptable salt, the solvate, the polymorph, the co-crystal, the stereoisomer or the isotopic compound thereof containing at least one isotopic atom with non-natural abundance.

As used herein, the term "subject" or "patient" refers to any animal to be treated or treated with the compound or the composition according to the embodiments of the present invention, preferably mammal, and most preferably human. The term "mammal" used herein includes any mammal. Examples of mammals include but not limited to cattle, horse, sheep, pig, cat, dog, mouse, rat, rabbit, guinea pig, monkey, human and the like, most preferably human. In an embodiment, the terms "treat" and "treating" refers to an improvement, prevention or reversal of a disease or condition or at least one of identifiable symptoms thereof, such as treating cancer by reducing or stabilizing the symptoms of the cancer or the condition. In another embodiment, "treat" or "treating" refers to an improvement, prevention or reversal of at least one measurable body parameter of a disease or condition which is being treated, but may not be identified in mammal. However, in another embodiment, the term "treat" or "treating" refers to slowing the progression of a disease or condition, in physical, such as stabilizing identifiable symptoms, or in physiological, such as stabilizing physical parameters, or in both. In another embodiment, the term "treat" or "treating" refers to delaying the development of a disease or symptom.

In some embodiments, the combination, pharmaceutical composition or kit is administered for a prevention purpose. As used herein, "prevent" or "preventing" refers to a reduction in a risk of obtaining a given disease or condition. In a preferred embodiment, the designated combination, pharmaceutical composition or kit is administered for a prevention purpose to a subject, such as a subject with family history or tendency of cancer or autoimmune disease.

As used herein, "therapeutically effective amount" refers to an amount of the compound or the composition (which is sought by researchers, veterinarians, physicians, or other clinicians) that can cause a biological or medical response in a tissue system, an animal or a person, which may include relieving symptoms of the disease or symptom which is being treated. In a preferred embodiment, the therapeutically effective amount is an amount which is enough to effectively treat, improve or prevent cancer, condition or undesirable angiogenesis.

The term "prophylactically effective amount" refers to an amount of the active compound or medicament (sought by researchers, veterinarians, physicians or other clinicians) that can inhibit the development of a disease in a subject. A prophylactically effective amount of the compound refers to an amount of the therapeutic agent used alone or in combination with other active compound, which can provide a therapeutic benefit for treating or preventing the disease, condition or disorder.

Each preferred conditions aforementioned can be combined randomly without departing from the common knowledge in the art thereby forming various preferred embodiments of the present invention.

Unless otherwise specified, the singular form of the term used herein, "a" or "an", also includes a plural meaning.

Unless otherwise specified, the term "or" or "and" used herein refers to "and/or".

Various publications, articles, and patents are cited or described herein. The citation or description of these references or the incorporation in their entirety or the discussion about them intends to illustrate the background of the present invention, but not to mean that the contents thereof form a part of the prior art of the present invention.

Unless defined otherwise, all technical and scientific terms used herein have the same meaning as commonly understood by an ordinary person skilled in the art to which this invention belongs. Otherwise, the meaning of certain terms used herein has the meaning set forth in this description.

In the present invention, the structures of the other therapeutic agent are as follows:

| | |
|---|---|
| ODM-201 (BAY-1841788) | 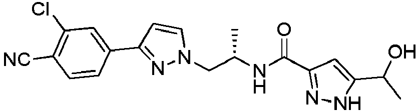 |
| Galeterone (TOK-001) | 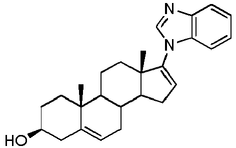 |
| Abiraterone acetate | 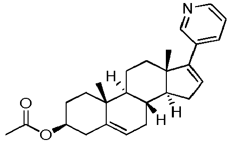 |
| Abiraterone | 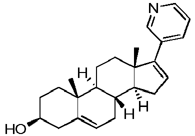 |
| Prednisone | 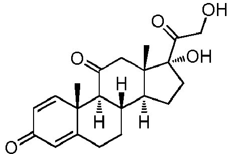 |

The reagents used in the invention are all commercially available. The compound of formula (I) and the androgen receptor pathway modulator in the invention may be prepared by people skilled in the art according to synthetic methods well known in the art, or readily synthesized according to the published literatures or patents, for example, the compound of formula (I) may be prepared according to the method published in WO9803502 , WO2008039489, WO2011100380 and so on; the androgen receptor pathway modulator may be prepared according to the method published in WO2013087004, WO2014190895, WO2011029392, CN201010120494.9, WO2011029392, WO2014036897 and so on.

The positive effect of the invention is that the combination of the invention can inhibit the growth of prostate cancer cells more effectively than the components used alone.

### Examples

The invention will be further illustrated by the following examples, but it should not be constructed that the invention is limited to the scope of the examples. The experimental methods that are not specified in details in the following examples are those according to conventional methods and conditions, or according to the product manuals.

### Effect embodiment 1 CTG cell proliferation assay

In vitro test of the inhibition effect of the benzoheteroyclic compound in combination with the androgen receptor pathway modulators or the benzoheteroyclic compound in combination with the androgen receptor pathway modulators and the other therapeutic agent on the prostate cancer cell proliferation.

Inhibition effect of the compound B001, the above androgen receptor pathway modulators, the above other therapeutic agents, alone or in combination with each other on the prostate cancer cell proliferation were tested on Vcap cells (androgen receptor (+) prostate cancer cells) (ATCC, catalogue number CRL-2876). The specific experimental operation was as follows: 5 × 10³ Vcap cells per well were inoculated into 96-well plates with transparent bottom and white wall (Corning, catalogue number CLS3903) containing the specific medium, and were cultured in a 37 °C, 5% CO₂ incubator for 24 hours. The tested compounds were prepared to a 150mM stocking solution with DMSO (Sigma, catalogue number 276855), diluted with culture medium to the desired concentrations (the final concentration of DMSO is 0.2%), and then added to each well, 2 wells/concentration, followed by being incubated in a 37 °C, 5% CO₂ incubator for 5 days. The tested compounds were used alone or in combination. The other therapeutic agents were: Abiraterone acetate (Selleck, catalogue number S2246), Galeterone (Selleck, catalogue number S2803), ODM-201 (Kangpu Biopharmaceuticals, Ltd.) or Prednisone (Selleck, catalogue number S1622). The concentration setting of each tested compound was shown in the following tables of experimental results. After that, 100 µl of CellTiter-Glo® cell viability assay reagent (Promega, catalogue number G7570) was added to each well and mixed on a vibrator for 10 minutes to induce cell lysis. The 96-well plate was placed at room temperature for 10 minutes, so as to stabilize its luminescence signal. A white bottom membrane was pasted on the bottom of the plate and the plate was tested using EnSpire. The data was processed by Graphpad/Prism and Calcusyn software to calculate the average cell proliferation inhibition rate or survival rate for each compound, and the specific experimental results were shown in Table 1(contains two parts: Table 1 Part I and Table 1 Part II) and Table 2(contains two parts: Table 2 Part I and Table 2 Part II).

Table 1(contains two parts: Table 1 Part I and Table 1 Part II) demonstrated the effects of the tested compound used alone or in combination with B001 or other therapeutic agents (including prednisone, Galeterone, abironate acetate, ODM-201) on the survival rate of Vcap cells. Because Table 1 contains a lot of information, Table 1 was divided into Table 1 Part I and Table 1 Part II.

Table 2(contains two parts: Table 2 Part I and Table 1 Part II) demonstrated the effects of the tested compounds used in combination with B001 and other therapeutic agents (including prednisone, Galeterone, abironate acetate, ODM-201) on the survival rate of Vcap cells. Because Table 2 contains a lot of information, Table 2 was divided into Table 2 Part I and Table 2 Part II.

**Table 1 Part I. Survival rate of Vcap cells: tested compound alone or in combination with B001 or other therapeutic agents (including prednisone, Galeterone, Abiraterone acetate, ODM-201)**

| Tested Compound | Tested Compound alone (1, 10µM) | | in combination with 1µM B001 | |
|---|---|---|---|---|
| | 10µM | 1µM | 10µM | 1µM |
| AR2-2 | 57.9% | 65.7% | 41.974% | 49.536% |
| AR2-1 | 61.3% | 74.0% | 46.508% | 54.401% |
| AR2-3 | 57.5% | 73.0% | 45.283% | 52.695% |
| AR2-4 | 41.0% | 73.5% | 32.946% | 53.165% |
| AR3-1 | 56.5% | 76.5% | 41.310% | 53.800% |
| AR3-4 | 66.1% | 77.0% | 47.983% | 52.699% |
| AR3-2 | 62.8% | 76.0% | 42.044% | 51.248% |
| AR3-3 | 68.7% | 83.5% | 45.721% | 59.220% |
| AR2-5 | 71.2% | 86.2% | 58.564% | 68.577% |
| AR1-1 | 61.1% | 74.5% | 43.631% | 51.992% |
| AR1-2 | 60.9% | 75.1% | 42.006% | 55.248% |
| B001 | 74.1% | 75.8% | | |
| Prednisone | 81.4% | 91.8% | | |
| Galeterone | 33.4% | 79.7% | | |
| Abiraterone acetate | 54.1% | 86.6% | | |
| ODM-201 | 67.6% | 67.9% | | |

**Table 1 Part II. Survival rate of Vcap cells: tested compound alone or in combination with B001 or other therapeutic agents (including prednisone, Galeterone, Abiraterone acetate, ODM-201)**

| Tested Compou nd | in combination with 1µM prednisone | | in combination with 1µM Galeterone | | in combination with 1 µM Abiraterone acetate | | in combination with 1µM ODM-201 | |
|---|---|---|---|---|---|---|---|---|
| | 10µ M | 1µM | 10µ M | 1µM | 10µ M | 1µM | 10µ M | 1µM |
| AR2-2 | 64.0 % | 79.5 % | 65.2 % | 70.9 % | 66.9 % | 74.3 % | 68.1 % | 75.0 % |
| AR2-1 | 58.5 % | 77.7 % | 58.3 % | 71.8 % | 58.3 % | 69.2 % | 61.4 % | 72.9 % |
| AR2-3 | 60.4 % | 78.6 % | 61.8 % | 75.8 % | 61.4 % | 74.1 % | 65.4 % | 79.9 % |
| AR2-4 | 42.3 % | 75.9 % | 38.4 % | 72.4 % | 42.4 % | 71.2 % | 42.3 % | 74.1 % |
| AR3-1 | 57.9 % | 81.6 % | 55.9 % | 74.3 % | 54.9 % | 72.8 % | 62.2 % | 76.1 % |
| AR3-4 | 69.1 % | 82.2 % | 68.1 % | 76.1 % | 64.5 % | 73.6 % | 68.4 % | 78.3 % |
| AR3-2 | 57.6 % | 77.1 % | 58.6 % | 73.4 % | 53.9 % | 70.8 % | 62.5 % | 71.7 % |
| AR3-3 | 60.3 % | 82.3 % | 62.2 % | 78.0 % | 57.5 % | 75.0 % | 64.5 % | 74.0 % |
| AR2-5 | 71.8 % | 89.0 % | 71.1 % | 81.6 % | 68.4 % | 80.0 % | 70.3 % | 75.7 % |
| AR1-1 | 62.2 % | 79.9 % | 66.6 % | 73.8 % | 60.6 % | 71.1 % | 64.2 % | 68.4 % |
| AR1-2 | 59.3 % | 78.3 % | 64.1 % | 76.9 % | 60.2 % | 71.2 % | 63.9 % | 73.3 % |

| | | | | | | | | |
|---|---|---|---|---|---|---|---|---|
| Notes: The results of Table 1 Part I and Table 1 Part II demonstrated that the effect of tested compound (AR2-2, AR2-1, AR2-3, AR2-4, AR3-1, AR3-4, AR3-2, AR3-3, AR2-5, AR1-1, AR1-2) in combination with B001 was outstanding, for example, according to Table 1 Part I, the cell survival rate was 65.7% when 1µM AR2-2 was used alone. The cell survival rate decreased to 49.536% when 1µM AR2-2 was used in combination with 1µM B001. However, the effect did not improve significantly when the tested compound (AR2-2, AR2-1, AR2-3, AR2-4, AR3-1, AR3-4, AR3-2, AR3-3, AR2-5, AR1-1, AR1-2) was used in combination with the other therapeutic agents (including prednisone, Galeterone, Abiraterone acetate, ODM-201). | | | | | | | | |

**Table 2 Part I. Survival rate of Vcap cells: tested compound in combination with B001 and other therapeutic agents (including prednisone, Galeterone, Abiraterone acetate, ODM-201)**

| Tested Compound | in combination with 1µM B001 and 1µM Prednisone | | in combination with 1µM B001 and 1µM Galeterone | | in combination with 1µM B001 and 1µM Abiraterone acetate | | in combination with 1µM B001 and 1µM ODM-201 | |
|---|---|---|---|---|---|---|---|---|
| | 10µM | 1µM | 10µM | 1µM | 10µM | 1µM | 10µM | 1µM |
| AR2-2 | 37.3 % | 50.5 % | 38.7 % | 43.6 % | 38.8 % | 47.4 % | 41.8 % | 47.4 % |
| AR2-1 | 48.4 % | 60.5 % | 48.7 % | 52.8 % | 47.1 % | 51.5 % | 51.0 % | 53.5 % |
| AR2-3 | 40.7 % | 51.7 % | 40.1 % | 45.2 % | 40.6 % | 47.0 % | 42.3 % | 47.4 % |
| AR2-4 | 34.2 % | 55.6 % | 30.7 % | 52.9 % | 33.3 % | 52.7 % | 33.9 % | 55.8 % |
| AR3-1 | 40.8 % | 57.9 % | 38.2 % | 48.4 % | 39.2 % | 49.6 % | 44.0 % | 51.4 % |
| AR3-4 | 44.0 % | 54.0 % | 43.7 % | 49.4 % | 41.9 % | 48.2 % | 46.9 % | 50.4 % |
| AR3-2 | 39.3 % | 56.4 % | 40.5 % | 47.9 % | 38.4 % | 46.9 % | 44.6 % | 50.3 % |
| AR3-3 | 49.1 % | 68.4 % | 48.1 % | 54.6 % | 43.6 % | 58.5 % | 50.3 % | 56.7 % |
| AR2-5 | 56.1 % | 67.2 % | 62.7 % | 68.6 % | 60.0 % | 66.6 % | 58.6 % | 57.3 % |
| AR1-1 | 40.0 % | 47.6 % | 48.6 % | 49.4 % | 44.1 % | 48.5 % | 46.9 % | 50.7 % |
| AR1-2 | 43.1 % | 55.1 % | 38.0 % | 49.9 % | 35.5 % | 47.3 % | 41.3 % | 46.8 % |

**Table 2 Part II. Survival rate of Vcap cells: tested compound in combination with B001 and other therapeutic agents (including prednisone, Galeterone, Abiraterone acetate, ODM-201)**

| Tested Compound | in combination with 1µM B001 and 1µM Prednisone and 1µM Galeterone | | in combination with 1µM B001 and 1µM Prednisone and 1µM Abiraterone acetate | | in combination with 1µM B001 and 1µM Prednisone and 1µM ODM-201 | |
|---|---|---|---|---|---|---|
| | 10µM | 1µM | 10µM | 1µM | 10µM | 1µM |
| AR2-2 | 35.2% | 46.2% | 35.6% | 46.4% | 40.5% | 46.9% |
| AR2-1 | 37.6% | 47.3% | 37.8% | 43.7% | 41.2% | 42.9% |
| AR2-3 | 51.9% | 56.0% | 51.9% | 57.7% | 54.5% | 61.9% |
| AR2-4 | 31.2% | 54.8% | 31.1% | 49.4% | 29.2% | 50.9% |
| AR3-1 | 37.9% | 54.5% | 37.5% | 55.8% | 42.6% | 52.7% |
| AR3-4 | 46.6% | 52.0% | 40.8% | 46.8% | 45.5% | 46.4% |
| AR3-2 | 45.7% | 56.2% | 43.4% | 57.0% | 49.8% | 56.3% |
| AR3-3 | 45.7% | 59.0% | 42.3% | 57.0% | 45.6% | 48.4% |
| AR2-5 | 58.4% | 71.5% | 57.3% | 72.2% | 59.6% | 65.6% |
| AR1-1 | 41.9% | 54.0% | 41.2% | 49.5% | 44.2% | 46.7% |
| AR1-2 | 44.4% | 58.5% | 44.5% | 59.5% | 50.8% | 62.6% |

| | | | | | | |
|---|---|---|---|---|---|---|
| Notes: The results of Table 2 Part I and Table 2 Part II demonstrated that the effect of tested compound (AR2-2, AR2-1, AR2-3, AR2-4, AR3-1, AR3-4, AR3-2, AR3-3, AR2-5, AR1-1, AR1-2) in combination with B001 was outstanding. When the tested compound was used in combination with B001 and the other therapeutic agent (including prednisone, Galeterone, Abiraterone acetate, ODM-201), the existence of the other therapeutic agent had no influence on the effect of the combination of the tested compound and B001. For example, the cell survival rate was 65.7% when 1µM AR2-2 was used alone (according to Table 1 Part I). The cell survival rate decreased to 49.536% when 1µM AR2-2 was used in combination with 1µM B001 (according to Table 1 Part I). The cell survival rate was 50.5% when 1 µM AR2-2 was used in combination with 1µM B001 and 1µM Prednisone. The cell survival rate was 43.6% when 1µM AR2-2 was used in combination with 1µM B001 and 1µM Galeterone, The cell survival rate was 47.4% when 1µM AR2-2 was used in combination with 1µM B001 and 1µM Abiraterone acetate. The cell survival rate was 47.4% when 1µM AR2-2 was used in combination with 1µM B001 and 1µM ODM-201. The cell survival rate was 46.2% when 1µM AR2-2 was used in combination with 1µM B001 and 1µM Prednisone and 1µM Galeterone. The cell survival rate was 46.4% when 1µM AR2-2 was used in combination with 1µM B001 and 1µM Prednisone and 1µM Abiraterone acetate. The cell survival rate was 46.9% when 1µM AR2-2 was used in combination with 1µM B001 and 1µM Prednisone and 1µM ODM-201. | | | | | | |

Although the embodiments of the invention were described above, it will be understood by people skilled in the art that these are just examples. Many changes and modifications can be made to these embodiments without departing from the principle and essence of the present invention. Therefore, the protection scope of the present invention is defined by the claims attached.

## Claims

1. A combination for the treatment of prostate cancer, comprising (i) one of a benzoheterocyclic compound, a pharmaceutically acceptable salt, a solvate, a polymorph, a co-crystal, a stereoisomer and an isotope compound thereof, and (ii) an androgen receptor pathway modulator;
wherein, the benzoheterocyclic compound is selected from the group consisting of: 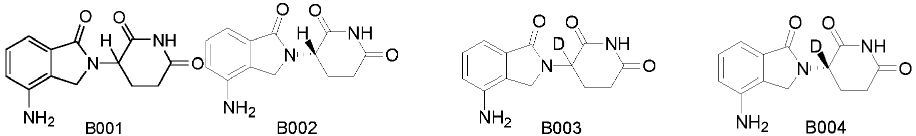 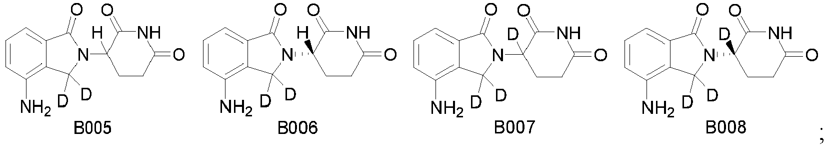 and
the androgen receptor pathway modulator is selected from:

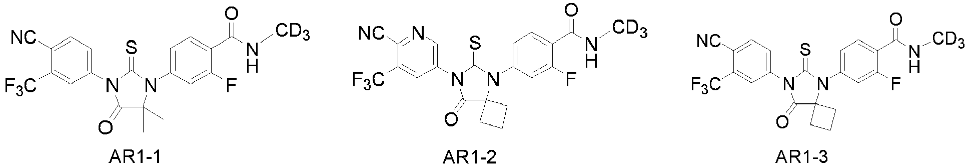

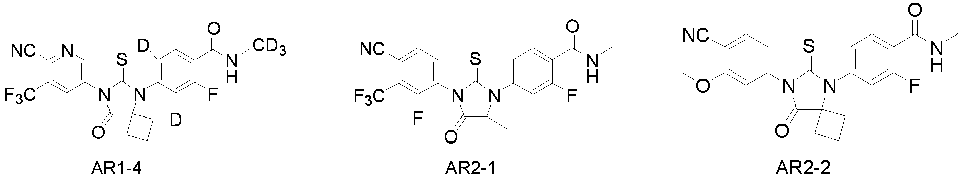

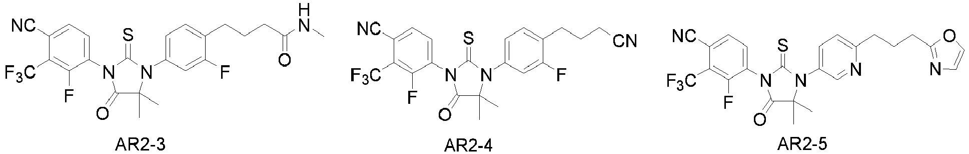

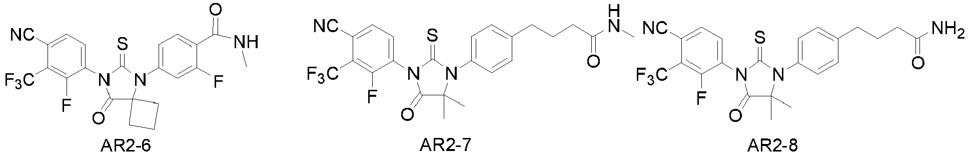

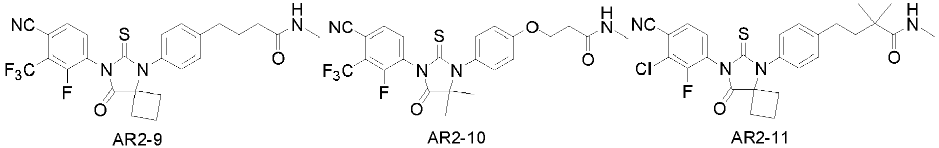

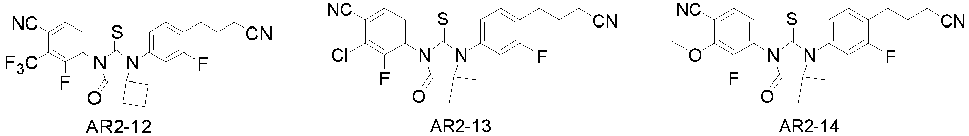

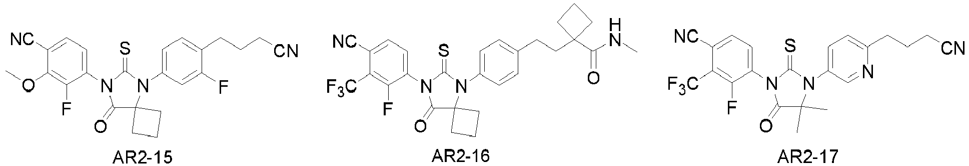

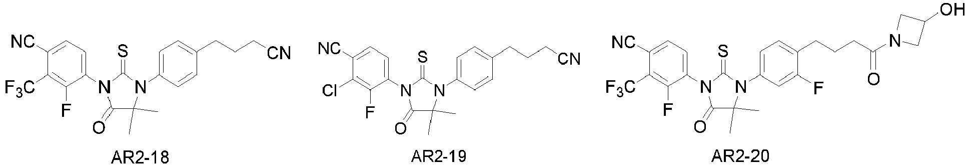

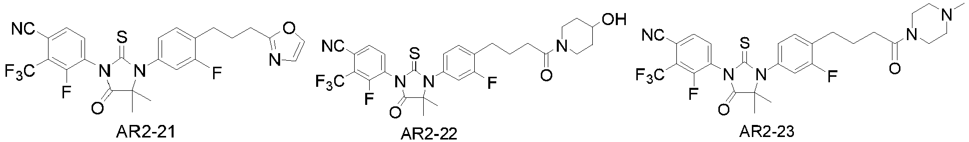

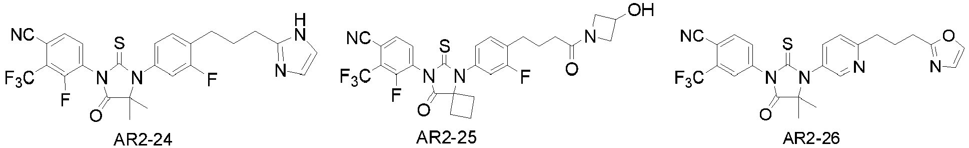

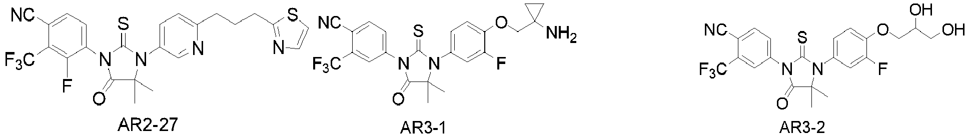

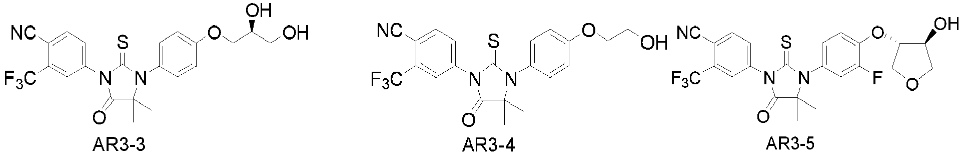

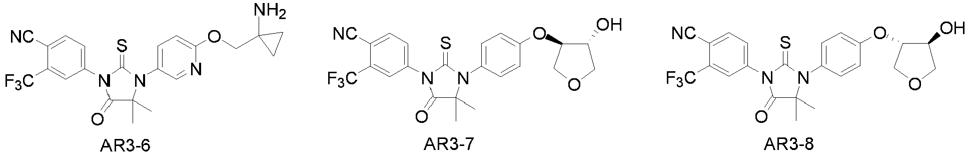

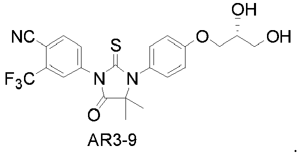

2. The combination for the treatment of prostate cancer according to claim 1, wherein, the androgen receptor pathway modulator is one of the following compounds: AR1-1, AR1-2, AR2-1, AR2-2, AR2-3, AR2-4, AR2-5, AR3-1, AR3-2, AR3-3, AR3-4.

3. The combination for the treatment of prostate cancer according to claim 2, wherein, the combination of benzoheterocyclic compound and the androgen receptor pathway modulator is one of the following compositions:
the combination of B001 and AR1-1, the combination of B001 and AR1-2, the combination of B001 and AR1-3, the combination of B001 and AR1-4, the combination of B001 and AR2-1, the combination of B001 and AR2-2, the combination of B001 and AR2-3, the combination of B001 and AR2-4, the combination of B001 and AR2-5, the combination of B001 and AR2-6, the combination of B001 and AR2-7, the combination of B001 and AR2-8, the combination of B001 and AR2-9, the combination of B001 and AR2-10, the combination of B001 and AR2-11, the combination of B001 and AR2-12, the combination of B001 and AR2-13, the combination of B001 and AR2-14, the combination of B001 and AR2-15, the combination of B001 and AR2-16, the combination of B001 and AR2-17, the combination of B001 and AR2-18, the combination of B001 and AR2-19, the combination of B001 and AR2-20, the combination of B001 and AR2-21, the combination of B001 and AR2-22, the combination of B001 and AR2-23, the combination of B001 and AR2-24, the combination of B001 and AR2-25, the combination of B001 and AR2-26, the combination of B001 and AR2-27, the combination of B001 and AR3-1, the combination of B001 and AR3-2, the combination of B001 and AR3-3, the combination of B001 and AR3-4, the combination of B001 and AR3-5, the combination of B001 and AR3-6, the combination of B001 and AR3-7, the combination of B001 and AR3-8, or the combination of B001 and AR3-9.

4. The combination for the treatment of prostate cancer according to claim 3, wherein, the combination of benzoheterocyclic compound and the androgen receptor pathway modulator is one of the following compositions:
B001 and AR1-1, B001 and AR1-2, B001 and AR2-1, B001 and AR2-2, B001 and AR2-3, B001 and AR2-4, B001 and AR2-5, B001 and AR3-1, B001 and AR3-2, B001 and AR3-3, B001 and AR3-4.

5. The combination for the treatment of prostate cancer according to any one of claims 1-4, wherein, the combination further comprises other therapeutic agent selected from one of Abiraterone, Abiraterone acetate, Galeterone, ODM201, Prednisone, Abiraterone and Prednisone, Abiraterone acetate and Prednisone, Galeterone and Prednisone, and, ODM201 and Prednisone.

6. The combination for the treatment of prostate cancer according to claim 5, wherein, the combination of benzoheterocyclic compound, the androgen receptor pathway modulator and the other therapeutic agent is any one of the following combinations:
B001 and AR1-1 and Abiraterone, B001 and AR1-2 and Abiraterone, B001 and AR2-1 and Abiraterone, B001 and AR2-2 and Abiraterone, B001 and AR2-3 and Abiraterone, B001 and AR2-4 and Abiraterone, B001 and AR2-5 and Abiraterone, B001 and AR3-1 and Abiraterone, B001 and AR3-2 and Abiraterone, B001 and AR3-3 and Abiraterone, B001 and AR3-4 and Abiraterone, B001 and AR1-1 and Abiraterone acetate, B001 and AR1-2 and Abiraterone acetate, B001 and AR2-1 and Abiraterone acetate, B001 and AR2-2 and Abiraterone acetate, B001 and AR2-3 and Abiraterone acetate, B001 and AR2-4 and Abiraterone acetate, B001 and AR2-5 and Abiraterone acetate, B001 and AR3-1 and Abiraterone acetate, B001 and AR3-2 and Abiraterone acetate, B001 and AR3-3 and Abiraterone acetate, B001 and AR3-4 and Abiraterone acetate, B001 and AR1-1 and Galeterone, B001 and AR1-2 and Galeterone, B001 and AR2-1 and Galeterone, B001 and AR2-2 and Galeterone, B001 and AR2-3 and Galeterone, B001 and AR2-4 and Galeterone, B001 and AR2-5 and Galeterone, B001 and AR3-1 and Galeterone, B001 and AR3-2 and Galeterone, B001 and AR3-3 and Galeterone, B001 and AR3-4 and Galeterone, B001 and AR1-1 and ODM201, B001 and AR1-2 and ODM201, B001 and AR2-1 and ODM201, B001 and AR2-2 and ODM201, B001 and AR2-3 and ODM201, B001 and AR2-4 and ODM201, B001 and AR2-5 and ODM201, B001 and AR3-1 and ODM201, B001 and AR3-2 and ODM201, B001 and AR3-3 and ODM201, B001 and AR3-4 and ODM201, B001 and AR1-1 and Prednisone, B001 and AR1-2 and Prednisone, B001 and AR2-1 and Prednisone, B001 and AR2-2 and Prednisone, B001 and AR2-3 and Prednisone, B001 and AR2-4 and Prednisone, B001 and AR2-5 and Prednisone, B001 and AR3-1 and Prednisone, B001 and AR3-2 and Prednisone, B001 and AR3-3 and Prednisone, B001 and AR3-4 and Prednisone, B001 and AR1-1 and Abiraterone and Prednisone, B001 and AR1-2 and Abiraterone and Prednisone, B001 and AR2-1 and Abiraterone and Prednisone, B001 and AR2-2 and Abiraterone and Prednisone, B001 and AR2-3 and Abiraterone and Prednisone, B001 and AR2-4 and Abiraterone and Prednisone, B001 and AR2-5 and Abiraterone and Prednisone, B001 and AR3-1 and Abiraterone and Prednisone, B001 and AR3-2 and Abiraterone and Prednisone, B001 and AR3-3 and Abiraterone and Prednisone, B001 and AR3-4 and Abiraterone and Prednisone, B001 and AR1-1 and Abiraterone acetate and Prednisone, B001 and AR1-2 and Abiraterone acetate and Prednisone, B001 and AR2-1 and Abiraterone acetate and Prednisone, B001 and AR2-2 and Abiraterone acetate and Prednisone, B001 and AR2-3 and Abiraterone acetate and Prednisone, B001 and AR2-4 and Abiraterone acetate and Prednisone, B001 and AR2-5 and Abiraterone acetate and Prednisone, B001 and AR3-1 and Abiraterone acetate and Prednisone, B001 and AR3-2 and Abiraterone acetate and Prednisone, B001 and AR3-3 and Abiraterone acetate and Prednisone, B001 and AR3-4 and Abiraterone acetate and Prednisone, B001 and AR1-1 and Galeterone and Prednisone, B001 and AR1-2 and Galeterone and Prednisone, B001 and AR2-1 and Galeterone and Prednisone, B001 and AR2-2 and Galeterone and Prednisone, B001 and AR2-3 and Galeterone and Prednisone, B001 and AR2-4 and Galeterone and Prednisone, B001 and AR2-5 and Galeterone and Prednisone, B001 and AR3-1 and Galeterone and Prednisone, B001 and AR3-2 and Galeterone and Prednisone, B001 and AR3-3 and Galeterone and Prednisone, B001 and AR3-4 and Galeterone and Prednisone, B001 and AR1-1 and ODM201 and Prednisone, B001 and AR1-2 and ODM201 and Prednisone, B001 and AR2-1 and ODM201 and Prednisone, B001 and AR2-2 and ODM201 and Prednisone, B001 and AR2-3 and ODM201 and Prednisone, B001 and AR2-4 and ODM201 and Prednisone, B001 and AR2-5 and ODM201 and Prednisone, B001 and AR3-1 and ODM201 and Prednisone, B001 and AR3-2 and ODM201 and Prednisone, B001 and AR3-3 and ODM201 and Prednisone, or B001 and AR3-4 and ODM201 and Prednisone.

7. A pharmaceutical composition, comprising the combination according to any one of claims 1-6 and one or more of the pharmaceutically acceptable excipients.

8. A kit, comprising a pharmaceutical composition A and a pharmaceutical composition B;
wherein, the pharmaceutical composition A comprises one of the benzoheterocyclic compound, the pharmaceutically acceptable salt, the solvate, the polymorph, the co-crystal, the stereoisomer and the isotope compound thereof according to any one of claims 1-4 and one or more of the pharmaceutically acceptable excipients;
the pharmaceutical composition B comprises the androgen receptor pathway modulator according to any one of claims 1-4 and one or more of the pharmaceutically acceptable excipients;
optionally, the kit further comprises a pharmaceutical composition C, wherein, the pharmaceutical composition C comprises the other therapeutic agent according to claim 5 or 6 and one or more of the pharmaceutically acceptable excipients.

9. The one of the benzoheterocyclic compound, the pharmaceutically acceptable salt, the solvate, the polymorph, the co-crystal, the stereoisomer and isotope compound thereof according to any one of claims 1-4 for use in the prevention and/or treatment of prostate cancer in combination with an androgen receptor pathway modulator according to any one of claims 1-4;
or the androgen receptor pathway modulator according to any one of claims 1-4 for use in the prevention and/or treatment of prostate cancer in combination with the one of the benzoheterocyclic compound, the pharmaceutically acceptable salt, the solvate, the polymorph, the co-crystal, the stereoisomer and the isotope compound according to any one of claims 1-4.

10. The androgen receptor pathway modulator for use according to claim 9, wherein the benzoheterocyclic compound, the pharmaceutically acceptable salt, the solvate, the polymorph, the co-crystal, the stereoisomer and the isotope compound thereof, and the androgen receptor pathway modulator are administered simultaneously or separately;
and/or, the prostate cancer is the castration-resistant prostate cancer.

11. The one of the benzoheterocyclic compound, the pharmaceutically acceptable salt, the solvate, the polymorph, the co-crystal, the stereoisomer and the isotope compound thereof according to any one of claims 1-6 for use in the prevention and/or treatment of prostate cancer in combination with the androgen receptor pathway modulator according to any one of claims 1-6 and the other therapeutic agent according to claim 5 or 6;
or, the androgen receptor pathway modulator according to any one of claims 1-6 for use in the prevention and/or treatment of prostate cancer in combination with the one of the benzoheterocyclic compound, the pharmaceutically acceptable salt, the solvate, the polymorph, the co-crystal, the stereoisomer and the isotope compound thereof according to any one of claims 1-6 and the other therapeutic agent according to claim 5 or 6;
or, the other therapeutic agent according to claim 5 or 6 for use in the prevention and/or treatment of prostate cancer in combination with the one of the benzoheterocyclic compound, the pharmaceutically acceptable salt, the solvate, the polymorph, the co-crystal, the stereoisomer and the isotope compound thereof according to any one of claims 1-6 and the androgen receptor pathway modulator according to any one of claims 1-6.

12. The androgen receptor pathway modulator for use according to claim 11, wherein the benzoheterocyclic compound, the pharmaceutically acceptable salt, the solvate, the polymorph, the co-crystal, the stereoisomer and the isotope compound thereof, the androgen receptor pathway modulator and the other therapeutic agent are administered simultaneously or separately;
and/or, the prostate cancer is the castration-resistant prostate cancer.

## Patentansprüche

1. Kombination zur Behandlung von Prostatakrebs, umfassend (i) eine benzoheterocyclische Verbindung, ein pharmazeutisch unbedenkliches Salz, ein Solvat, ein Polymorph, einen Cokristall, ein Stereoisomer oder eine Isotopverbindung davon und (ii) einen Androgenrezeptorweg-Modulator;
wobei die benzoheterocyclische Verbindung aus der Gruppe bestehend aus 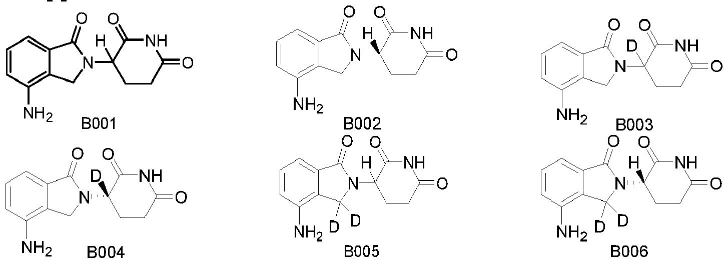 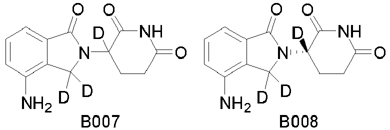 ausgewählt ist und
der Androgenrezeptorweg-Modulator aus 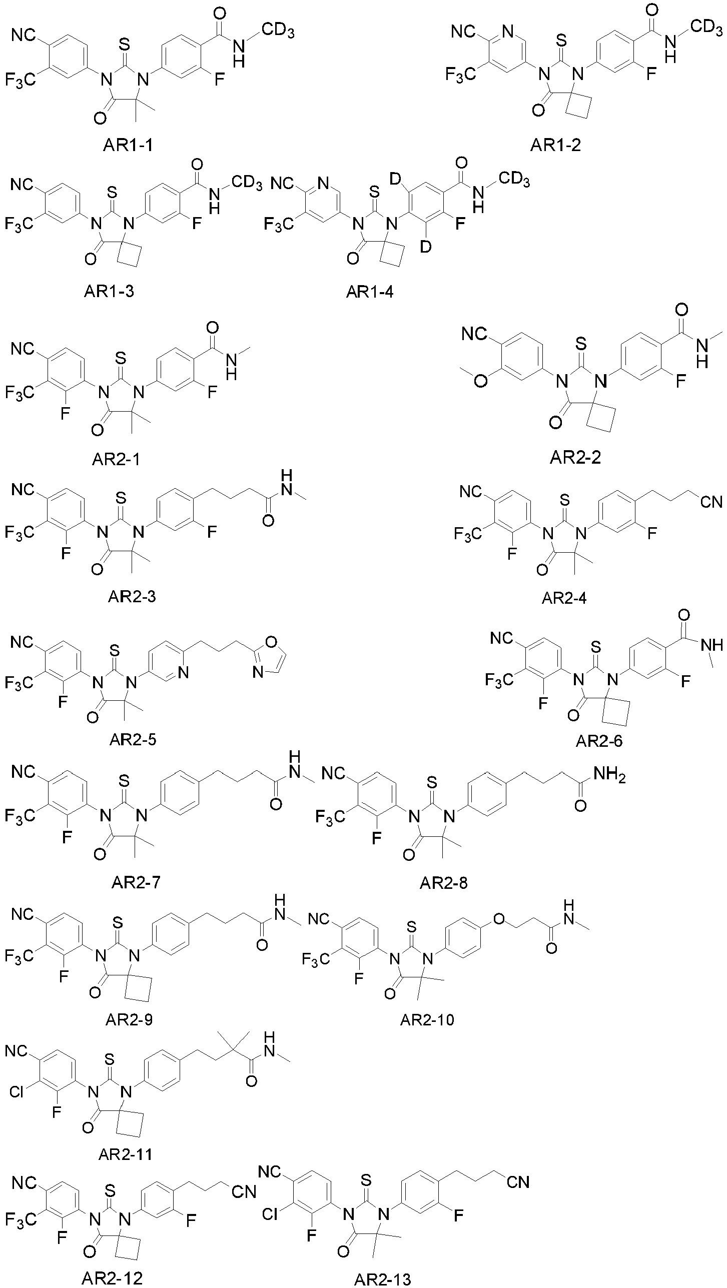 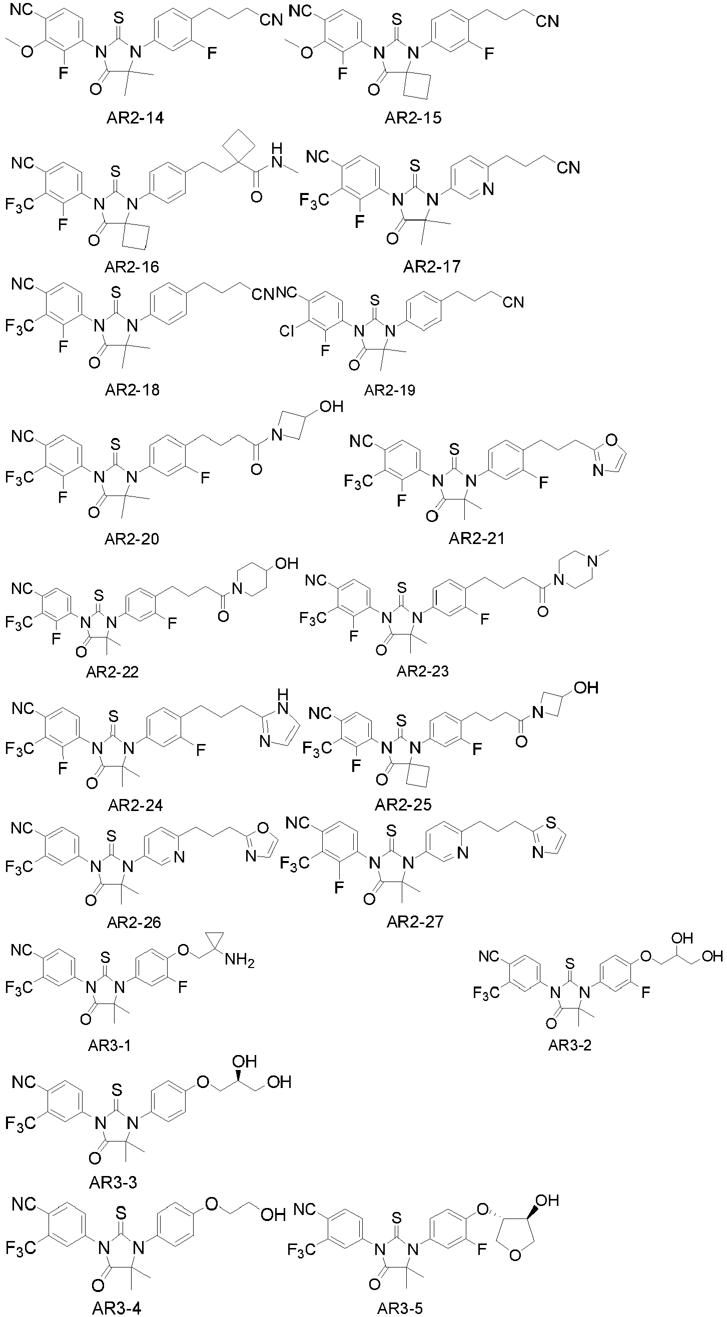 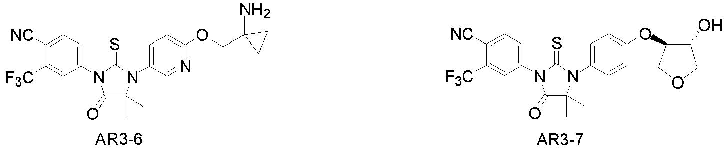 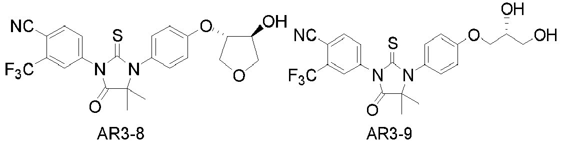 ausgewählt ist.

2. Kombination zur Behandlung von Prostatakrebs nach Anspruch 1, wobei es sich bei dem Androgenrezeptorweg-Modulator um eine der folgenden Verbindungen handelt: AR1-1, AR1-2, AR2-1, AR2-2, AR2-3, AR2-4, AR2-5, AR3-1, AR3-2, AR3-3, AR3-4.

3. Kombination zur Behandlung von Prostatakrebs nach Anspruch 2, wobei es sich bei der Kombination von benzoheterocyclischer Verbindung und dem Androgenrezeptorweg-Modulator um eine der folgenden Zusammensetzungen handelt:
die Kombination von B001 und AR1-1, die Kombination von B001 und AR1-2, die Kombination von B001 und AR1-3, die Kombination von B001 und AR1-4, die Kombination von B001 und AR2-1, die Kombination von B001 und AR2-2, die Kombination von B001 und AR2-3, die Kombination von B001 und AR2-4, die Kombination von B001 und AR2-5, die Kombination von B001 und AR2-6, die Kombination von B001 und AR2-7, die Kombination von B001 und AR2-8, die Kombination von B001 und AR2-9, die Kombination von B001 und AR2-10, die Kombination von B001 und AR2-11, die Kombination von B001 und AR2-12, die Kombination von B001 und AR2-13, die Kombination von B001 und AR2-14, die Kombination von B001 und AR2-15, die Kombination von B001 und AR2-16, die Kombination von B001 und AR2-17, die Kombination von B001 und AR2-18, die Kombination von B001 und AR2-19, die Kombination von B001 und AR2-20, die Kombination von B001 und AR2-21, die Kombination von B001 und AR2-22, die Kombination von B001 und AR2-23, die Kombination von B001 und AR2-24, die Kombination von B001 und AR2-25, die Kombination von B001 und AR2-26, die Kombination von B001 und AR2-27, die Kombination von B001 und AR3-1, die Kombination von B001 und AR3-2, die Kombination von B001 und AR3-3, die Kombination von B001 und AR3-4, die Kombination von B001 und AR3-5, die Kombination von B001 und AR3-6, die Kombination von B001 und AR3-7, die Kombination von B001 und AR3-8 oder die Kombination von B001 und AR3-9.

4. Kombination zur Behandlung von Prostatakrebs nach Anspruch 3, wobei es sich bei der Kombination von benzoheterocyclischer Verbindung und dem Androgenrezeptorweg-Modulator um eine der folgenden Zusammensetzungen handelt:
B001 und AR1-1, B001 und AR1-2, B001 und AR2-1, B001 und AR2-2, B001 und AR2-3, B001 und AR2-4, B001 und AR2-5, B001 und AR3-1, B001 und AR3-2, B001 und AR3-3, B001 und AR3-4.

5. Kombination zur Behandlung von Prostatakrebs nach einem der Ansprüche 1-4, wobei die Kombination ferner ein anderes therapeutisches Mittel umfasst, das aus einem von Abirateron, Abirateronacetat, Galeteron, ODM201, Prednison, Abirateron und Prednison, Abirateronacetat und Prednison, Galeteron und Prednison und ODM201 und Prednison ausgewählt ist.

6. Kombination zur Behandlung von Prostatakrebs nach Anspruch 5, wobei es sich bei der Kombination von benzoheterocyclischer Verbindung, dem Androgenrezeptorweg-Modulator und dem anderen therapeutischen Mittel um eine der folgenden Kombinationen handelt:
B001 und AR1-1 und Abirateron, B001 und AR1-2 und Abirateron, B001 und AR2-1 und Abirateron, B001 und AR2-2 und Abirateron, B001 und AR2-3 und Abirateron, B001 und AR2-4 und Abirateron, B001 und AR2-5 und Abirateron, B001 und AR3-1 und Abirateron, B001 und AR3-2 und Abirateron, B001 und AR3-3 und Abirateron, B001 und AR3-4 und Abirateron, B001 und AR1-1 und Abirateronacetat, B001 und AR1-2 und Abirateronacetat, B001 und AR2-1 und Abirateronacetat, B001 und AR2-2 und Abirateronacetat, B001 und AR2-3 und Abirateronacetat, B001 und AR2-4 und Abirateronacetat, B001 und AR2-5 und Abirateronacetat, B001 und AR3-1 und Abirateronacetat, B001 und AR3-2 und Abirateronacetat, B001 und AR3-3 und Abirateronacetat, B001 und AR3-4 und Abirateronacetat, B001 und AR1-1 und Galeteron, B001 und AR1-2 und Galeteron, B001 und AR2-1 und Galeteron, B001 und AR2-2 und Galeteron, B001 und AR2-3 und Galeteron, B001 und AR2-4 und Galeteron, B001 und AR2-5 und Galeteron, B001 und AR3-1 und Galeteron, B001 und AR3-2 und Galeteron, B001 und AR3-3 und Galeteron, B001 und AR3-4 und Galeteron, B001 und AR1-1 und ODM201, B001 und AR1-2 und ODM201, B001 und AR2-1 und ODM201, B001 und AR2-2 und ODM201, B001 und AR2-3 und ODM201, B001 und AR2-4 und ODM201, B001 und AR2-5 und ODM201, B001 und AR3-1 und ODM201, B001 und AR3-2 und ODM201, B001 und AR3-3 und ODM201, B001 und AR3-4 und ODM201, B001 und AR1-1 und Prednison, B001 und AR1-2 und Prednison, B001 und AR2-1 und Prednison, B001 und AR2-2 und Prednison, B001 und AR2-3 und Prednison, B001 und AR2-4 und Prednison, B001 und AR2-5 und Prednison, B001 und AR3-1 und Prednison, B001 und AR3-2 und Prednison, B001 und AR3-3 und Prednison, B001 und AR3-4 und Prednison, B001 und AR1-1 und Abirateron und Prednison, B001 und AR1-2 und Abirateron und Prednison, B001 und AR2-1 und Abirateron und Prednison, B001 und AR2-2 und Abirateron und Prednison, B001 und AR2-3 und Abirateron und Prednison, B001 und AR2-4 und Abirateron und Prednison, B001 und AR2-5 und Abirateron und Prednison, B001 und AR3-1 und Abirateron und Prednison, B001 und AR3-2 und Abirateron und Prednison, B001 und AR3-3 und Abirateron und Prednison, B001 und AR3-4 und Abirateron und Prednison, B001 und AR1-1 und Abirateronacetat und Prednison, B001 und AR1-2 und Abirateronacetat und Prednison, B001 und AR2-1 und Abirateronacetat und Prednison, B001 und AR2-2 und Abirateronacetat und Prednison, B001 und AR2-3 und Abirateronacetat und Prednison, B001 und AR2-4 und Abirateronacetat und Prednison, B001 und AR2-5 und Abirateronacetat und Prednison, B001 und AR3-1 und Abirateronacetat und Prednison, B001 und AR3-2 und Abirateronacetat und Prednison, B001 und AR3-3 und Abirateronacetat und Prednison, B001 und AR3-4 und Abirateronacetat und Prednison , B001 und AR1-1 und Galeteron und Prednison, B001 und AR1-2 und Galeteron und Prednison, B001 und AR2-1 und Galeteron und Prednison, B001 und AR2-2 und Galeteron und Prednison, B001 und AR2-3 und Galeteron und Prednison, B001 und AR2-4 und Galeteron und Prednison, B001 und AR2-5 und Galeteron und Prednison, B001 und AR3-1 und Galeteron und Prednison, B001 und AR3-2 und Galeteron und Prednison, B001 und AR3-3 und Galeteron und Prednison, B001 und AR3-4 und Galeteron und Prednison, B001 und AR1-1 und ODM201 und Prednison, B001 und AR1-2 und ODM201 und Prednison, B001 und AR2-1 und ODM201 und Prednison, B001 und AR2-2 und ODM201 und Prednison, B001 und AR2-3 und ODM201 und Prednison, B001 und AR2-4 und ODM201 und Prednison, B001 und AR2-5 und ODM201 und Prednison, B001 und AR3-1 und ODM201 und Prednison, B001 und AR3-2 und ODM201 und Prednison, B001 und AR3-3 und ODM201 und Prednison oder B001 und AR3-4 und ODM201 und Prednison handelt.

7. Pharmazeutische Zusammensetzung, umfassend die Kombination nach einem der Ansprüche 1-6 und einen oder mehrere pharmazeutisch unbedenkliche Hilfsstoffe.

8. Kit, umfassend eine pharmazeutische Zusammensetzung A und eine pharmazeutische Zusammensetzung B;
wobei die pharmazeutische Zusammensetzung A die benzoheterocyclische Verbindung, das pharmazeutisch unbedenkliche Salz, das Solvat, das Polymorph, den Cokristall, das Stereoisomer oder die Isotopverbindung davon nach einem der Ansprüche 1-4 und einen oder mehrere pharmazeutisch unbedenkliche Hilfsstoffe umfasst;
die pharmazeutische Zusammensetzung B den Androgenrezeptorweg-Modulator nach einem der Ansprüche 1-4 und einen oder mehrere pharmazeutisch unbedenkliche Hilfsstoffe umfasst; gegebenenfalls wobei das Kit ferner eine pharmazeutische Zusammensetzung C umfasst, wobei die pharmazeutische Zusammensetzung C das andere therapeutische Mittel nach Anspruch 5 oder 6 und einen oder mehrere pharmazeutisch unbedenkliche Hilfsstoffe umfasst.

9. Benzoheterocyclische Verbindung, pharmazeutisch unbedenkliches Salz, Solvat, Polymorph, Cokristall, Stereoisomer oder Isotopverbindung davon nach einem der Ansprüche 1-4 zur Verwendung bei der Prävention und/oder Behandlung von Prostatakrebs in Kombination mit einem Androgenrezeptorweg-Modulator nach einem der Ansprüche 1-4;
oder Androgenrezeptorweg-Modulator nach einem der Ansprüche 1-4 zur Verwendung bei der Prävention und/oder Behandlung von Prostatakrebs in Kombination mit der benzoheterocyclischen Verbindung, dem pharmazeutisch unbedenklichen Salz, dem Solvat, dem Polymorph, dem Cokristall, dem Stereoisomer oder der Isotopverbindung nach einem der Ansprüche 1-4.

10. Androgenrezeptorweg-Modulator zur Verwendung nach Anspruch 9, wobei die benzoheterocyclische Verbindung, das pharmazeutisch unbedenkliche Salz, das Solvat, das Polymorph, der Cokristall, das Stereoisomer oder die Isotopverbindung davon und der Androgenrezeptorweg-Modulator gleichzeitig oder separat verabreicht werden
und/oder es sich bei dem Prostatakrebs um kastrationsresistenten Prostatakrebs handelt.

11. Benzoheterocyclische Verbindung, pharmazeutisch unbedenkliches Salz, Solvat, Polymorph, Cokristall, Stereoisomer oder Isotopverbindung davon nach einem der Ansprüche 1-6 zur Verwendung bei der Prävention und/oder Behandlung von Prostatakrebs in Kombination mit dem Androgenrezeptorweg-Modulator nach einem der Ansprüche 1-6 und dem anderen therapeutischen Mittel nach Anspruch 5 oder 6
oder Androgenrezeptorweg-Modulator nach einem der Ansprüche 1-6 zur Verwendung bei der Prävention und/oder Behandlung von Prostatakrebs in Kombination mit der benzoheterocyclischen Verbindung, dem pharmazeutisch unbedenklichen Salz, dem Solvat, dem Polymorph, dem Cokristall, dem Stereoisomer oder der Isotopverbindung davon nach einem der Ansprüche 1-6 und dem anderen therapeutischen Mittel nach Anspruch 5 oder 6
oder anderes therapeutisches Mittel nach Anspruch 5 oder 6 zur Verwendung bei der Prävention und/oder Behandlung von Prostatakrebs in Kombination mit der benzoheterocyclischen Verbindung, dem pharmazeutisch unbedenklichen Salz, dem Solvat, dem Polymorph, dem Cokristall, dem Stereoisomer oder der Isotopverbindung davon nach einem der Ansprüche 1-6 und dem Androgenrezeptorweg-Modulator nach einem der Ansprüche 1-6.

12. Androgenrezeptorweg-Modulator zur Verwendung nach Anspruch 11, wobei die benzoheterocyclische Verbindung, das pharmazeutisch unbedenkliche Salz, das Solvat, das Polymorph, der Cokristall, das Stereoisomer oder die Isotopverbindung davon, der Androgenrezeptorweg-Modulator und das andere therapeutische Mittel gleichzeitig oder separat verabreicht werden
und/oder es sich bei dem Prostatakrebs um kastrationsresistenten Prostatakrebs handelt.

## Revendications

1. Combinaison pour le traitement du cancer de la prostate, comprenant (i) l'un parmi un composé benzohétérocyclique, un sel pharmaceutiquement acceptable, un solvate, une forme polymorphe, un cocristal, un stéréoisomère et un composé isotopique de celui-ci, et (ii) un modulateur de la voie des récepteurs des androgènes ;
dans laquelle, le composé benzohétérocyclique est choisi dans le groupe constitué de : 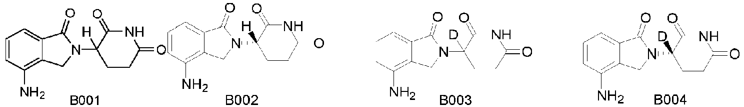 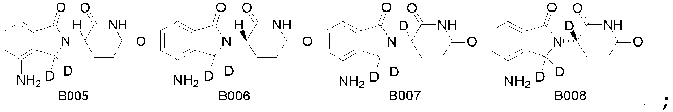 et
le modulateur de la voie des récepteurs des androgènes est choisi parmi :

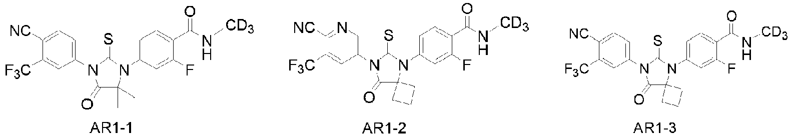

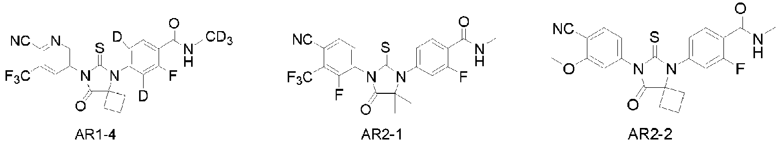

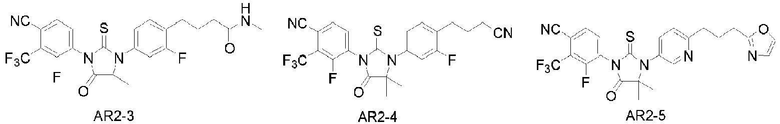

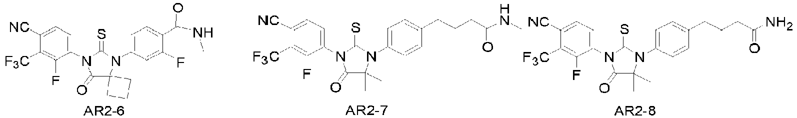

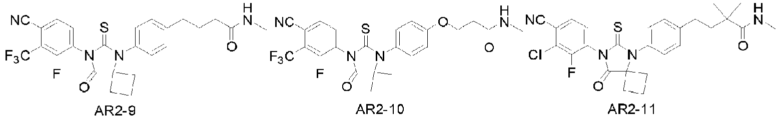

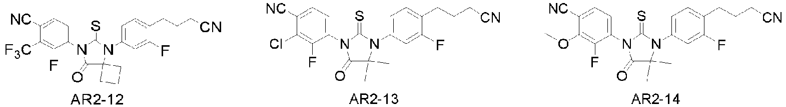

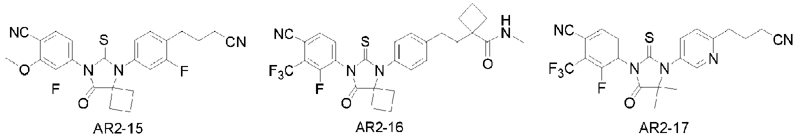

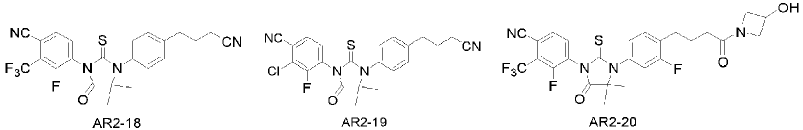

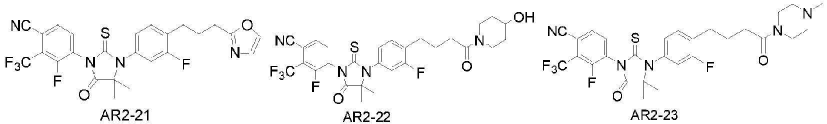

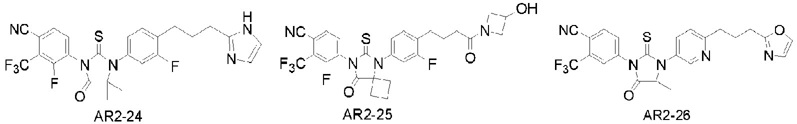

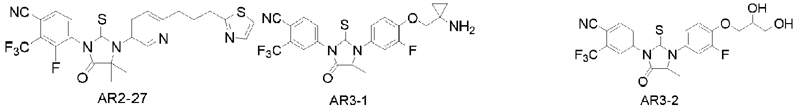

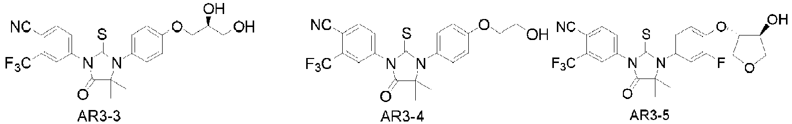

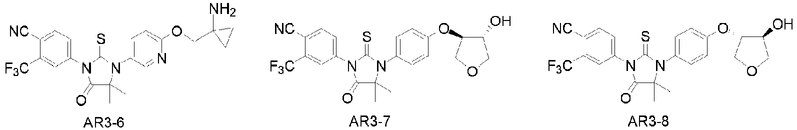

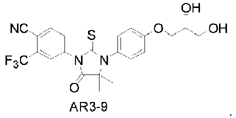

2. Combinaison pour le traitement du cancer de la prostate selon la revendication 1, où, le modulateur de la voie des récepteurs des androgènes est l'un des composés suivants : AR1-1, AR1-2, AR2-1, AR2-2, AR2-3, AR2-4, AR2-5, AR3-1, AR3-2, AR3-3, AR3-4.

3. Combinaison pour le traitement du cancer de la prostate selon la revendication 2, où, la combinaison de composé benzohétérocyclique et le modulateur de la voie des récepteurs des androgènes est l'une des compositions suivantes :
la combinaison de B001 et AR1-1, la combinaison de B001 et AR1-2, la combinaison de B001 et AR1-3, la combinaison de B001 et AR1-4, la combinaison de B001 et AR2-1, la combinaison de B001 et AR2-2, la combinaison de B001 et AR2-3, la combinaison de B001 et AR2-4, la combinaison de B001 et AR2-5, la combinaison de B001 et AR2-6, la combinaison de B001 et AR2-7, la combinaison de B001 et AR2-8, la combinaison de B001 et AR2-9, la combinaison de B001 et AR2-10, la combinaison de B001 et AR2-11, la combinaison de B001 et AR2-12, la combinaison de B001 et AR2-13, la combinaison de B001 et AR2-14, la combinaison de B001 et AR2-15, la combinaison de B001 et AR2-16, la combinaison de B001 et AR2-17, la combinaison de B001 et AR2-18, la combinaison de B001 et AR2-19, la combinaison de B001 et AR2-20, la combinaison de B001 et AR2-21, la combinaison de B001 et AR2-22, la combinaison de B001 et AR2-23, la combinaison de B001 et AR2-24, la combinaison de B001 et AR2-25, la combinaison de B001 et AR2-26, la combinaison de B001 et AR2-27, la combinaison de B001 et AR3-1, la combinaison de B001 et AR3-2, la combinaison de B001 et AR3-3, la combinaison de B001 et AR3-4, la combinaison de B001 et AR3-5, la combinaison de B001 et AR3-6, la combinaison de B001 et AR3-7, la combinaison de B001 et AR3-8, ou la combinaison de B001 et AR3-9.

4. Combinaison pour le traitement du cancer de la prostate selon la revendication 3, où, la combinaison du composé benzohétérocyclique et du modulateur de la voie des récepteurs des androgènes est l'une des compositions suivantes :
B001 et AR1-1, B001 et AR1-2, B001 et AR2-1, B001 et AR2-2, B001 et AR2-3, B001 et AR2-4, B001 et AR2-5, B001 et AR3-1, B001 et AR3-2, B001 et AR3-3, B001 et AR3-4.

5. Combinaison pour le traitement du cancer de la prostate selon l'une quelconque des revendications 1 à 4, où, la combinaison comprend en outre un autre agent thérapeutique choisi parmi l'un de : abiratérone, acétate d'abiratérone, galétérone, ODM201, prednisone, abiratérone et prednisone, acétate d'abiratérone et prednisone, galétérone et prednisone, et ODM201 et prednisone.

6. Combinaison pour le traitement du cancer de la prostate selon la revendication 5, où, la combinaison du composé benzohétérocyclique, du modulateur de la voie des récepteurs des androgènes et de l'autre agent thérapeutique est l'une quelconque des combinaisons suivantes :
B001 et AR1-1 et abiratérone, B001 et AR1-2 et abiratérone, B001 et AR2-1 et abiratérone, B001 et AR2-2 et abiratérone, B001 et AR2-3 et abiratérone, B001 et AR2-4 et abiratérone, B001 et AR2-5 et abiratérone, B001 et AR3-1 et abiratérone, B001 et AR3-2 et abiratérone, B001 et AR3-3 et abiratérone, B001 et AR3-4 et abiratérone, B001 et AR1-1 et acétate d'abiratérone, B001 et AR1-2 et acétate d'abiratérone, B001 et AR2-1 et acétate d'abiratérone, B001 et AR2-2 et acétate d'abiratérone, B001 et AR2-3 et acétate d'abiratérone, B001 et AR2-4 et acétate d'abiratérone, B001 et AR2-5 et acétate d'abiratérone, B001 et AR3-1 et acétate d'abiratérone, B001 et AR3-2 et acétate d'abiratérone, B001 et AR3-3 et acétate d'abiratérone, B001 et AR3-4 et acétate d'abiratérone, B001 et AR1-1 et galétérone, B001 et AR1-2 et galétérone, B001 et AR2-1 et galétérone, B001 et AR2-2 et galétérone, B001 et AR2-3 et galétérone, B001 et AR2-4 et galétérone, B001 et AR2-5 et galétérone, B001 et AR3-1 et galétérone, B001 et AR3-2 et galétérone, B001 et AR3-3 et galétérone, B001 et AR3-4 et galétérone, B001 et AR1-1 et ODM201, B001 et AR1-2 et ODM201, B001 et AR2-1 et ODM201, B001 et AR2-2 et ODM201, B001 et AR2-3 et ODM201, B001 et AR2-4 et ODM201, B001 et AR2-5 et ODM201, B001 et AR3-1 et ODM201, B001 et AR3-2 et ODM201, B001 et AR3-3 et ODM201, B001 et AR3-4 et ODM201, B001 et AR1-1 et prednisone, B001 et AR1-2 et prednisone, B001 et AR2-1 et prednisone, B001 et AR2-2 et prednisone, B001 et AR2-3 et prednisone, B001 et AR2-4 et prednisone, B001 et AR2-5 et prednisone, B001 et AR3-1 et prednisone, B001 et AR3-2 et prednisone, B001 et AR3-3 et prednisone, B001 et AR3-4 et prednisone, B001 et AR1-1 et abiratérone et prednisone, B001 et AR1-2 et abiratérone et prednisone, B001 et AR2-1 et abiratérone et prednisone, B001 et AR2-2 et abiratérone et prednisone, B001 et AR2-3 et abiratérone et prednisone, B001 et AR2-4 et abiratérone et prednisone, B001 et AR2-5 et abiratérone et prednisone, B001 et AR3-1 et abiratérone et prednisone, B001 et AR3-2 et abiratérone et prednisone, B001 et AR3-3 et abiratérone et prednisone, B001 et AR3-4 et abiratérone et prednisone, B001 et AR1-1 et acétate d'abiratérone et prednisone, B001 et AR1-2 et acétate d'abiratérone et prednisone, B001 et AR2-1 et acétate d'abiratérone et prednisone, B001 et AR2-2 et acétate d'abiratérone et prednisone, B001 et AR2-3 et acétate d'abiratérone et prednisone, B001 et AR2-4 et acétate d'abiratérone et prednisone, B001 et AR2-5 et acétate d'abiratérone et prednisone, B001 et AR3-1 et acétate d'abiratérone et prednisone, B001 et AR3-2 et acétate d'abiratérone et prednisone, B001 et AR3-3 et acétate d'abiratérone et prednisone, B001 et AR3-4 et acétate d'abiratérone et prednisone, B001 et AR1-1 et galétérone et prednisone, B001 et AR1-2 et galétérone et prednisone, B001 et AR2-1 et galétérone et prednisone, B001 et AR2-2 et galétérone et prednisone, B001 et AR2-3 et galétérone et prednisone, B001 et AR2-4 et galétérone et prednisone, B001 et AR2-5 et galétérone et prednisone, B001 et AR3-1 et galétérone et prednisone, B001 et AR3-2 et galétérone et prednisone, B001 et AR3-3 et galétérone et prednisone, B001 et AR3-4 et galétérone et prednisone, B001 et AR1-1 et ODM201 et prednisone, B001 et AR1-2 et ODM201 et prednisone, B001 et AR2-1 et ODM201 et prednisone, B001 et AR2-2 et ODM201 et prednisone, B001 et AR2-3 et ODM201 et prednisone, B001 et AR2-4 et ODM201 et prednisone, B001 et AR2-5 et ODM201 et prednisone, B001 et AR3-1 et ODM201 et prednisone, B001 et AR3-2 et ODM201 et prednisone, B001 et AR3-3 et ODM201 et prednisone, ou B001 et AR3-4 et ODM201 et prednisone.

7. Composition pharmaceutique, comprenant la combinaison selon l'une quelconque des revendications 1 à 6 et un ou plusieurs des excipients pharmaceutiquement acceptables.

8. Trousse, comprenant une composition pharmaceutique A et une composition pharmaceutique B ; dans laquelle, la composition pharmaceutique A comprend l'un parmi le composé benzohétérocyclique, le sel pharmaceutiquement acceptable, le solvate, la forme polymorphe, le cocristal, le stéréoisomère et le composé isotopique de celui-ci selon l'une quelconque des revendications 1 à 4 et un ou plusieurs des excipients pharmaceutiquement acceptables ; la composition pharmaceutique B comprend le modulateur de la voie des récepteurs des androgènes selon l'une quelconque des revendications 1 à 4 et un ou plusieurs des excipients pharmaceutiquement acceptables ; facultativement, la trousse comprend en outre une composition pharmaceutique C, dans laquelle, la composition pharmaceutique C comprend l'autre agent thérapeutique selon la revendication 5 ou 6 et un ou plusieurs des excipients pharmaceutiquement acceptables.

9. L'un parmi le composé benzohétérocyclique, le sel pharmaceutiquement acceptable, le solvate, la forme polymorphe, le cocristal, le stéréoisomère et le composé isotopique de celui-ci selon l'une quelconque des revendications 1 à 4 pour utilisation dans la prévention et/ou le traitement du cancer de la prostate en combinaison avec un modulateur de la voie des récepteurs des androgènes selon l'une quelconque des revendications 1 à 4 ;
ou le modulateur de la voie des récepteurs des androgènes selon l'une quelconque des revendications 1 à 4 pour utilisation dans la prévention et/ou le traitement du cancer de la prostate en combinaison avec l'un parmi le composé benzohétérocyclique, le sel pharmaceutiquement acceptable, le solvate, la forme polymorphe, le cocristal, le stéréoisomère et le composé isotopique selon l'une quelconque des revendications 1 à 4.

10. Modulateur de la voie des récepteurs des androgènes pour utilisation selon la revendication 9, où le composé benzohétérocyclique, le sel pharmaceutiquement acceptable, le solvate, la forme polymorphe, le cocristal, le stéréoisomère et le composé isotopique de celui-ci, et le modulateur de la voie des récepteurs des androgènes sont administrés simultanément ou séparément ;
et/ou, le cancer de la prostate est le cancer de la prostate résistant à la castration.

11. L'un parmi le composé benzohétérocyclique, le sel pharmaceutiquement acceptable, le solvate, la forme polymorphe, le cocristal, le stéréoisomère et le composé isotopique de celui-ci selon l'une quelconque des revendications 1 à 6 pour utilisation dans la prévention et/ou le traitement du cancer de la prostate en combinaison avec le modulateur de la voie des récepteurs des androgènes selon l'une quelconque des revendications 1 à 6 et l'autre agent thérapeutique selon la revendication 5 ou 6 ;
ou, le modulateur de la voie des récepteurs des androgènes selon l'une quelconque des revendications 1 à 6 pour utilisation dans la prévention et/ou le traitement du cancer de la prostate en combinaison avec l'un parmi le composé benzohétérocyclique, le sel pharmaceutiquement acceptable, le solvate, la forme polymorphe, le cocristal, le stéréoisomère et le composé isotopique de celui-ci selon l'une quelconque des revendications 1 à 6 et l'autre agent thérapeutique selon la revendication 5 ou 6 ;
ou, l'autre agent thérapeutique selon la revendication 5 ou 6 pour utilisation dans la prévention et/ou le traitement du cancer de la prostate en combinaison avec l'un parmi le composé benzohétérocyclique, le sel pharmaceutiquement acceptable, le solvate, la forme polymorphe, le cocristal, le stéréoisomère et le composé isotopique de celui-ci selon l'une quelconque des revendications 1 à 6 et le modulateur de la voie des récepteurs des androgènes selon l'une quelconque des revendications 1 à 6.

12. Modulateur de la voie des récepteurs des androgènes pour utilisation selon la revendication 11, où le composé benzohétérocyclique, le sel pharmaceutiquement acceptable, le solvate, la forme polymorphe, le cocristal, le stéréoisomère et le composé isotopique de celui-ci, le modulateur de la voie des récepteurs des androgènes et l'autre agent thérapeutique sont administrés simultanément ou séparément ;
et/ou, le cancer de la prostate est le cancer de la prostate résistant à la castration.
